# EUROPEAN PATENT SPECIFICATION

(11) **EP 4 135 745 B1**
(45) Date of publication and mention of the grant of the patent: **07.05.2025**
(21) Application number: 21717933.2
(22) Date of filing: 16.04.2021
(51) Int. Cl.: A61K 38/17, A61K 8/00, A61P 17/02, A61P 17/14, A61K 8/64, A61Q 19/00

(54) **SCD83 FOR WOUND HEALING, HAIR GROWTH, AND SKIN AND HAIR CARE**
SCD83 FÜR WUNDHEILUNG, HAARWACHSTUM UND HAUT- UND HAARPFLEGE
SCD83 POUR LA CICATRISATION DES PLAIES, LA CROISSANCE DES CHEVEUX ET LES SOINS DE LA PEAU ET DES CHEVEUX

(30) Priority: 16.04.2020 EP 20169899
(43) Date of publication of application: 22.02.2023
(73) Proprietor: Friedrich-Alexander-Universität Erlangen-Nürnberg, 91054 Erlangen (DE)
(72) Inventor: STEINKASSERER, Alexander, 91352 Hallerndorf (DE); ZINSER, Elisabeth, 91056 Erlangen (DE); ROYZMAN, Dmytro, 90542 Eckental (DE)
(74) Representative: dompatent von Kreisler Selting Werner - Partnerschaft von Patent- und Rechtsanwälten mbB
(86) International application number: PCT/EP2021/059923
(87) International publication number: WO 2021/209607

(56) References cited:
- WO-A1-2013/006505
- US-A1- 2011 182 903
- ZIDUO LI ET AL: "CD83: Activation Marker for Antigen Presenting Cells and Its Therapeutic Potential", FRONTIERS IN IMMUNOLOGY, vol. 10, 1 January 2019 (2019-01-01), pages 1312, XP055731281, DOI: 10.3389/fimmu.2019.01312
- JOE M. HORVATINOVICH ET AL: "Soluble CD83 Inhibits T Cell Activation by Binding to the TLR4/MD-2 Complex on CD14 + Monocytes", THE JOURNAL OF IMMUNOLOGY, vol. 198, no. 6, 13 February 2017 (2017-02-13), US, pages 2286 - 2301, XP055731233, ISSN: 0022-1767, DOI: 10.4049/jimmunol.1600802

## Description

Provided herein are specific soluble forms of a member of the CD83 family of proteins (sCD83) for use in wound closure of cutaneous wounds, including hard-to-heal wounds of elderly-, diabetic-, or patients under immunosuppressant medication. Further provided are said sCD83 for use in therapeutic hair growth after physical stress/medication and/or disease, and cosmetic non-therapeutic methods for the acceleration of hair growth and for hair care comprising the topical application of said sCD83.

### Background of the Invention

Injuries are not just a painful event, but also disrupt the protective function of the skin-barrier and enhance the susceptibility to infections. Therefore a fast wound healing is crucial for maintaining the physical integrity of the skin. However aging and health conditions are described to hamper the regenerative capacities of skin tissue, e.g. diabetes mellitus (Swift, M.E. et al., J Invest Dermatol 117:1027-1035 (2001); Goodson, W.H.3rd and Hung, T.K., J Surg Res 22:221-227 (1977)). Smoking and malnutrition contribute to wound healing complications (Janis, J.E. and Harrison, B., Plast Reconstr Surg 133:199e-207e (2014)). Furthermore, wound healing is negatively affected by the medication with immunosuppressive drugs and glucocorticoid steroids, used upon transplantations or the treatment of autoimmune disorders. Finally, radiation therapy leads to ineffective wound repair, thereby prolonging the phase of pain and susceptibility (Guo, S. and Dipietro, L.A.., J Dent Res 89:219-229 (2010)). Thus, enhanced wound healing is of major medical interests to avoid discomfort and post invasive complications.

Mechanistically, wound healing is a complex multifactorial event, which can be divided into four major phases: (I) hemostasis, (II) inflammation, (III) reepithelization and (IV) remodeling, which are characterized by distinct cell compositions and cytokine expression profiles (Eming, S.A. et al., Sci Transl Med 6:265sr266 (2014)). Upon injury, platelets attenuate blood flow by coagulation and induce the inflammatory phase by the release of α-granules (Zarbock, A. et al., Blood Rev 21:99-111 (2007)). Neutrophils, classically activated macrophages and lymphocytes are recruited to the site of lesion to sterilize the wound area and promote inflammation. Proinflammatory cytokines are the key regulators and dominate within the wound tissue, since the depletion of various proinflammatory cytokines (e.g. TNFα and IL-6) during wound healing result in an impaired resolution of cutaneous wounds (Ritsu, M. et al., J Dermatology & Dermatologic Surgery 21:14-19 (2017); Lin, Z.Q. et al., J Leukoc Biol 73:713-721 (2003)). Subsequently to TLR-stimulation and the phagocytosis of apoptotic cells (mainly neutrophils), macrophages undergo a phenotypic switch towards tissue-repair and/or tolerogenic alternatively activated macrophages (AAM) (Daley, J.M. et al., J Immunol 174:2265-2272 (2005)). Anti-inflammatory mediators as IL-10 and growth factors like TGF-β and VEGF, which are released by AAMs play a crucial role for the transition towards the third phase, i.e. the proliferation phase (Mosser, D.M. and Edwards, J.P., Nat Rev Immunol 8:958-969 (2008)). The secretion of VEGF induces and stabilizes both angiogenesis and lymph-angiogenesis to ensure a sufficient supply of nutrients and cellular compounds (e.g. keratinocytes and fibroblasts) to restore damaged tissue by enhanced migration and proliferation. Wound healing is completed when tissue integrity and function is restored within the remodeling phase.

Analogous to cutaneous wounding, intestinal epithelial injuries as typically observed in patients suffering from inflammatory bowel diseases (IBD), such as Ulcerative Colitis (UC) and Crohn's Disease (CD), cause physical stress by breaking the intestinal immune homeostasis and inducing severe intestinal inflammation. It is currently assumed that in these pathologies initial trigger for the loss of intestinal barrier integrity are environmental factors (Galvez, J., ISRN Inflamm 2014, 928461 (2014); Kiesler, P. et al., Cell Mol Gastroenterol Hepatol 1:154-170 (2015)). Furthermore, it is well documented that the invasion of luminal organisms or their products into the mucosa trigger effector cytokine driven chronic and recurrent inflammation of the gastrointestinal (GI) tract (Galvez, J., ISRN Inflamm 2014, 928461 (2014); Kiesler, P. et al., Cell Mol Gastroenterol Hepatol 1:154-170 (2015); Nenci, A. et al., Nature 446:557-561 (2007); Friswell, M. et al., Gut Liver 4:295-306 (2010)). Extensive research also revealed that one reason for this is a defect in the mucosal regulation of pro- and anti-inflammatory processes as a response to unknown components of the normal microflora (Galvez, J., ISRN Inflamm 2014, 928461 (2014)). During the course of inflammation, infiltrating immune cells continue to damage the inflamed tissue, as they are more susceptible to bacterial antigens than the resident macrophages and epithelial cells (Nenci, A. et al., Nature 446:557-561 (2007); Sartor, R.B., Nat Clin Pract Gastroenterol Hepatol 3:390-407 (2006); Reaves, T.A. et al., Mem Inst Oswaldo Cruz 100(Suppl 1):191-198 (2005)). Additionally, microbial factors and deranged epithelial cell functions can support these excessive immunologic responses. Therefore, the pathogenetic concept of IBD has changed from the classical autoimmune disease to a complex barrier disorder with a progressive and disruptive character (Kiesler, P. et al., Cell Mol Gastroenterol Hepatol 1:154-170 (2015); Neurath, M., Nat Rev Gastroenterol Hepatol 14:688 (2017)).

Intestinal wound healing involves various time-coordinated events that are initiated immediately upon mucosal injury. Within a few minutes after the injury, a necessary and crucial step for wound healing, the so-called epithelial restitution, occurs. This process stimulates the migration of intestinal epithelial cells (IECs) into the injured tissue and thus acts independently to processes of cell proliferation (Neurath, M.F., Mucosal Immunol 7:6-19 (2014)). Within the epithelial restitution process, various molecules such as the transforming growth factor α (TGF-α), interleukin-1 beta (IL-1β), the epidermal growth factor (EGF), interferon gamma (IFN-γ) (Dignass, A.U. and Podolsky, D.K., Gastroenterology 105:1323-1332 (1993)) as well as GTPases of the Rho family (Hall, A., Biochem Soc Trans 33:891-895 (2005)), have already been identified to have a beneficial regulatory effect by promoting the expression of TGF-β1 in the IECs (Neurath, M.F., Mucosal Immunol 7:6-19 (2014)). Interestingly, subsequent regulatory processes of IEC proliferation and differentiation are strongly dependent on molecules such as IL-6, TNF-α and platelet-derived growth factor (PDGF), as these mechanisms need to be strictly controlled. This following phase starts within hours or days after initial disruption and is essential for the mucosal wound closure. In this regard, different growth factors and cytokines, such as IL-6 and IL-22 as well as Toll-like-receptor (TLR) ligands are sufficient to boost cell proliferation, expansion and survival in IECs by the induction of specific transcription factors (TFs), whereas pro-inflammatory cytokines, such as IFN-γ and TNFs are essential to counter-regulate these signaling cascades. In addition, different TLRs, such as TLR2, TLR4 and TLR9 have been identified by multiple studies to reflect decisive regulators of wound healing (Jin, F.Y. et al., Cell 88:417-426 (1997)).

The CD83 molecule, once just known as a maturation marker expressed on mature dendritic cells (DCs), has revealed promising immunomodulatory properties in the context of prevention and treatment of autoimmune disease and transplantation models (Royzman, D. et al., Front Immunol 10:633 (2019); Zinser, E. et al., J Exp Med 200:345-351 (2004); Ge, W. et al., Transplantation 90:1145-1156 (2010); Lan, Z. et al., Transplantation 90:1286-1293 (2010); WO04/046182).

Nowadays, several immune cell types, including T- and B cells, regulatory T cells (Tregs), macrophages and others have been described to express CD83 (Li, Z. et al., Front Immunol 10:1312 (2019), and Grosche, L. et al., Front Immunol doi: 10.3389/fimmu.2020.00721 (2020)), which reportedly is crucial for cell function. For instance, Doebbeler et al. have reported that CD83 expression is essential for Treg development and stability and that mice with a Treg-specific conditional CD83 knockout are hampered in their tolerogenic function and provide rather an inflammatory milieu (Doebbeler, M. et al., JCI Insight 3 (2018)). On DCs, the absence of CD83 resulted in a significantly increased expression of co-stimulatory molecules CD25 and OX40L and subsequently enhanced T cell activation (Wild, A.B. et al., JCI Insight 4 (2019)). Noteworthy, both mouse models revealed an aggravated EAE-severity along with a delayed resolution of neuro-inflammation (Doebbeler, M. et al., JCI Insight 3 (2018); Wild, A.B. et al., JCI Insight 4 (2019)). However, the underlying mechanism(s) remained unknown until Horvatinovitsch et al. described that sCD83 binds to the MD2-TLR4 complex and represents an interaction partner for sCD83 on PBMCs. This interaction, in the long run, inhibits the pro-inflammatory TLR-mediated signaling cascade and induces an alternative anti-inflammatory environment (Horvatinovich, J.M. et al., J Immunol 198:2286-2301 (2017)). Interestingly, upon sCD83 administration, IL-6 expression is strongly induced in monocytes, which as mentioned above is crucial for cutaneous wounds. Further Bock et al. demonstrated that the systemic, and therapeutically even more important, also the topical application of sCD83 enhances organ acceptance in high risk transplantation models in a Treg- an IDO-mediated mechanism (Bock, F. et al., J Immunol 191:1965-1975 (2013)). Indeed, transplantations are often accompanied by severe tissue damage and wounding, where complications can massively affect the course of acceptance and healing. Medication with immunosuppressives prevent organ rejection on the one side, but lead to impaired wound healing on the other side, affecting transplant acceptance and function in the long-term (Roine, E. et al., Transplant Proc 42:2542-2546 (2010)). In this context, US2011/0182903 and WO2013/006505 disclose the use of sCD83, wherby in WO2013/006505 the sCD83 is a Fc-CD83 fusion protein, and CD83 antibodies, respectively, for the treatment of autoimmune diseases. WO2013/006505, in its Examples 1-9, also also describes the antiinflammatory effect of a Fc-CD83 fusion protein and of sCD83 proteins in the context of autoimmune diseases. Besides the reportedly beneficial effects on transplantation experiments, whereby soluble CD83 was reported to induce tolerogenic DCs that prevent e.g. cardiac allograft rejection, and the treatment of autoimmune diseases, the aim of this study was to elucidate the therapeutic potential of sCD83 regarding wound healing using two mouse models. This included (i) the DSS-induced colitis model and (ii) studies on mechanically induced skin injuries.

In addition, data regarding the impact of sCD83 on hair regeneration are provided. Although hair loss is not a life-threatening disorder, it can strikingly affect patient's quality of life and is associated with higher prevalence of anxiety and depression (Baghestani, S. et al., Dermatol Reports 7:6063 (2015)). Effluvium can be divided into a variety of medical conditions and triggers, e.g. scarring alopecia, androgenetic alopecia, alopecia areata and a few more (Phillips, T.G. et al., Am Fam Physician 96:371-378 (2017)). The most prevalent form of hair loss (androgenetic alopecia) is associated with genetic predisposition and hormonal imbalance, which subsequently result in a shortened phase of hair growth (anagen), while the length of telogen (hair follicle quiescence) is rather prolonged, thereby reducing hair length with every single hair cycle ((Ellis, J.A. et al., Expert Rev Mol Med 4:1-11 (2002); Courtois, M. et al., Skin Pharmacol 7:84-89 (1994)). Moreover, strong medication, such as chemotherapy and immunosuppressants (e.g. methotrexate) cause severe effluvium in a great number of patients (Trueb, R.M., Skin Therapy Lett 15:5-7 (2010); Lukasik, A. et al., Pol Merkur Lekarski 46:77-79 (2019)).

To date, medical treatment of hair loss is limited to two FDA-approved drugs, i.e. minoxidil and finasteride (Lee, S.W. et al., J Drugs Dermatol 17:457-463 (2018)) with annual sales of 860 million US$ in 2019 for minoxidil (https://www.marketwatch.com/press-release/at-47-cagr-minoxidil-market-size-is-expected-to-reach-1130-million-by-2024-2019-04-29). It is hypothesized that the effects of minoxidil are based on its enhanced vasodilatory properties and the induction of VEGF in dermal papilla cells, which subsequently increase local vascularization and the nutrient supply (Messenger, A.G. and Rundegren, J., Br J Dermatol 150:186-194 (2004)). In contrast, the administration of finasteride increases the anagen to telogen ratio, by reversing the abbreviation of the anagen phase in alopecia patients (Shapiro, J., and Kaufman, K.D., J Investig Dermatol Symp Proc 8:20-23 (2003)). Mechanistically, finasteride prevents the formation of dihydrotestosterone, by selectively antagonizing the Steroid-5α-Reduktase type 2 (Finn, D.A. et al., CNS Drug Rev 12:53-76 (2006)). Herein, the role of dihydrotestosterone is highly controversial, since it stimulates the hair growth of the facial and genital area, while promoting hair loss on the scalp in patients with genetically-determined sensitivity of the hair follicles (Urysiak-Czubatka, I. et al., Postepy Dermatol Alergol 31:207-215 (2014)). The reduction of dihydrotestosterone levels by finasteride slows the progression of male androgenetic alopecia along with a partial hair regrowth in two third of male patients (Van Neste, D. et al., Br J Dermatol 143:804-810 (2000)). However, according to various studies, finasteride-therapy is associated with severe side effects as erectile dysfunction, male infertility and gynecomastia (Traish, A.M. et al., J Sex Med 8:872-884 (2011); Ramot, Y. et al., Int J Trichology 1:27-29 (2009)). However, both medications (minoxidil and finasteride) require life-long administration and do not completely reverse preceding effluvium, but rather prevent further baldness, if the medication is successful at all.

### Summary of the Invention

The present invention is as defined in the claims. Wound healing, both cutaneous and intestinal, is a highly delicate and multifactorial process, where slight changes may result in delayed wound closure, scar formation or fibrosis. Generally, wound healing can be divided into four major phases i.e. hemostasis, inflammation, reepithelization and remodeling, each characterized by a unique cell- and cytokine profile, respectively. Particularly, medication by e.g. corticosteroids or cytokine-inhibiting biologicals, such as anti-IL-6 and anti-TNFα antibody therapies, suppress the inflammatory response and adversely affect the progression of wound closure, thereby providing discomfort and enhanced vulnerability to infections. Thus, new and more effective therapeutic wound care strategies are mandatory. In sharp contrast to commonly used immunosuppressive agents, the soluble CD83 (sCD83) molecule has immunomodulatory properties and has been shown to be very efficacious in numerous transplantation models, including renal-, skin-, heart- and high-risk cornea transplantation, without any notable adverse effects to date. Herein, the administration of sCD83 induced an immunomodulatory environment, characterized by the induction of regulatory T cells (Tregs), in an indoleamine-2,3-dioxygenase (IDO) dependent manner. Since among other cells, Tregs play a crucial role in wound healing processes, we investigated the role of sCD83 during these events. Strikingly, here we show for the first time, the systemic and, most notably also topical administration of sCD83 promotes/ improves wound healing in a pre-clinical skin wound model. In addition, using the DSS-induced colitis model, we could improve wound healing also in this specific intestinal wound healing model. In both cases, wound closure was significantly accelerated in sCD83-treated mice. Mechanistically, in sCD83 treated animals, the temporary expression of proinflammatory cytokines, e.g. IL-1β, IFNy, IL-17A and TNFα, was strongly increased during the inflammation phase, and reduced at later stages, which correlates with a better wound healing. Interestingly, the cellular composition within the skin, revealed an increased accumulation of B- and T cells in cutaneous wounds of sCD83-treated mice, which as reported, contribute to better cell survival and proliferation of e.g. fibroblasts, during reepithelization and remodeling phase. Thus, the data presented in the following provide the basis for future treatment options of e.g. hard-to-heal wounds of elderly-, diabetic-, or patients under immunosuppressant medication by application of sCD83. The data further provide the promotion of hair growth after physical stress and/or depilation by application of systemic sCD83, and also by its topical application. The invention thus provides:
(1) A soluble form of a member of the CD83 family of proteins consisting of the extracellular domain of the CD83 protein with the amino acid residues 20 to 144 of SEQ ID NO:2 (hereinafter shortly referred to as "sCD83 protein" or "sCD83"), a functional derivative of said sCD83 protein having up to 10 amino acids attached to the C- or N-terminus of said sCD83 protein and/or having up to 10 amino acids of said sCD83 protein substituted, a dimeric or trimeric form of said sCD83 protein, or a dimeric or trimeric form of the functional derivative of said sCD83 protein, for use in wound closure of cutaneous wounds;
(2) a soluble form of a member of the CD83 family of proteins consisting of the extracellular domain of the CD83 protein with the amino acid residues 20 to 144 of SEQ ID NO:2 (sCD83 protein), a functional derivative of having up to 10 amino acids attached to the C- or N-terminus of said sCD83 protein and/or having up to 10 amino acids of said sCD83 substituted, a dimeric or trimeric form of said sCD83 protein, or a dimeric or trimeric form of the functional derivative of said sCD83 protein, for use in therapeutic hair growth after physical stress/medication and/or disease;
(3) a composition comprising a soluble form of a member of the CD83 family of proteins consisting of the extracellular domain of the CD83 protein with the amino acid residues 20 to 144 of SEQ ID NO:2 (sCD83 protein), a functional derivative of said sCD83 protein having up to 10 amino acids attached to the C- or N-terminus of said sCD83 protein and/or having up to 10 amino acids of said sCD83 protein substituted, a dimeric or trimeric form of said sCD83 protein, or a dimeric or trimeric form of the functional derivative of said sCD83 protein, for use in therapeutic hair growth after physical stress/medication and/or disease; and
(4) a cosmetic non-therapeutic method for the acceleration of hair growth and for hair care, which method comprising systemically or topically applying a composition comprising a soluble form of a member of the CD83 family of proteins consisting of the extracellular domain of the CD83 protein with the amino acid residues 20 to 144 of SEQ ID NO:2 (sCD83 protein), a functional derivative of said sCD83 protein having up to 10 amino acids attached to the C- or N-terminus of said sCD83 protein and/or having up to 10 amino acids of said sCD83 protein substituted, a dimeric or trimeric form of said sCD83 protein, or a dimeric or trimeric form of the functional derivative of said sCD83 protein to the human or animal skin or hair.

### Short Description of the Figures

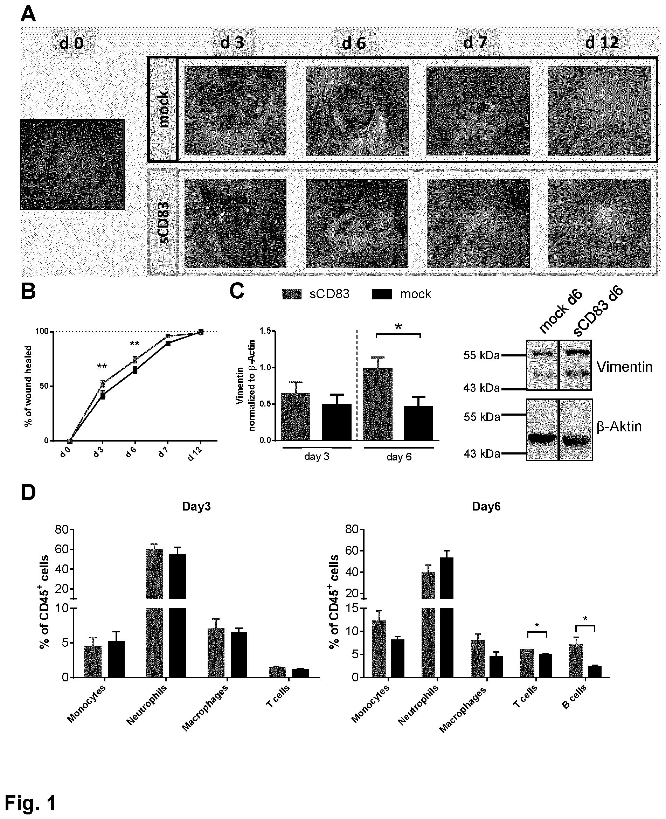

Figure 1 - Cutaneous wound healing is enhanced in systemically sCD83-treated mice: (A) Wound lesions were induced using 6 mm punches and the wound area was assessed by caliper at indicated time points. (B) Percentage of wound closure was set relatively to the day 0 value, with sCD83 and mock n ≥ 12. (C) Vimentin expression was analyzed on day 3 and day 6 by WB and quantified relatively to β-actin controls using ImageJ with n ≥ 10 for each group (left-hand side). Representative vimentin and β-actin bands from a WB-analysis of sCD83- and mock-treated day 6 skin samples (right-hand side). (D) Flow cytometric analyses of cellular composition within the wound margins on day 3 and day 6 with n=3 for each group. Data are illustrated as mean ± SEM and statistically analyzed using two-way ANOVA (B) and Mann-Whitney (C) and unpaired multiple t-test (D). Asterisks mark statistically significant difference (*p < 0.05 and **p < 0.01). The absence of asterisks indicates that there is no statistical significance.

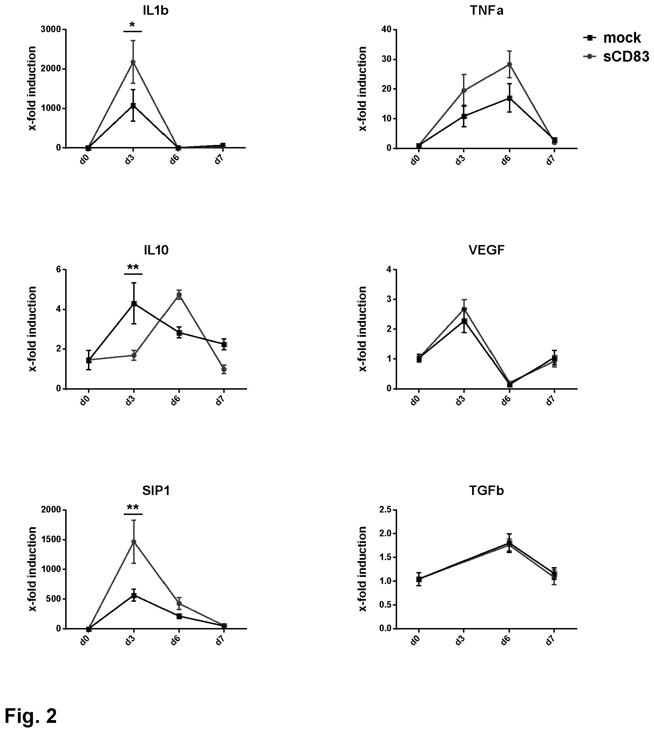

Figure 2 - sCD83 modulates the inflammatory and wound healing phase: RNA was isolated from wound tissue at indicated time points and used for qPCR-analyses. n ≥ 5 for each group. Data are illustrated as mean ± SEM and statistically analyzed using two-way ANOVA. Asterisks mark statistically significant difference (*p < 0.05 and **p < 0.01). The absence of asterisks indicates that there is no statistical significance.

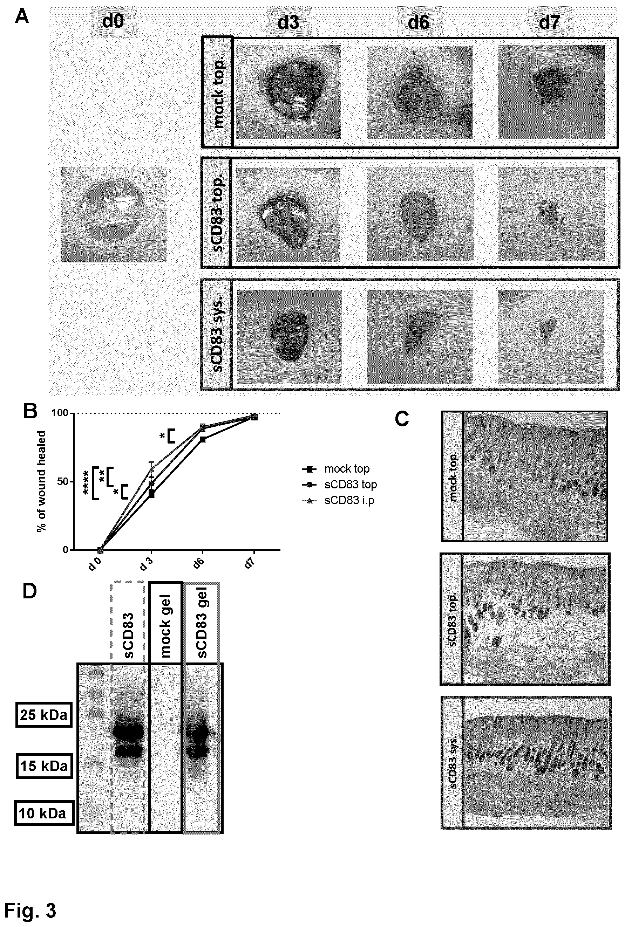

Figure 3 - Topic sCD83 application induces local wound healing: Wound lesions were set using 6 mm punches and diameters were measured by caliper at indicated time points. (A+B) Percentage of wound closure was set relatively to the day 0 value, with each group n=10. (C) Representative histological analyses of day 7 samples. (D) Western blot analyses of sCD83-hydrogel matrices after 10 days at 4 °C, demonstrate the stability of the sCD83 protein. Data are illustrated as mean ± SEM and statistically analyzed using two-way ANOVA (B). Asterisks mark statistically significant difference (*p < 0.05, **p < 0.01, ***p < 0.001, and ****p < 0.0001). The absence of asterisks indicates that there is no statistical significance.

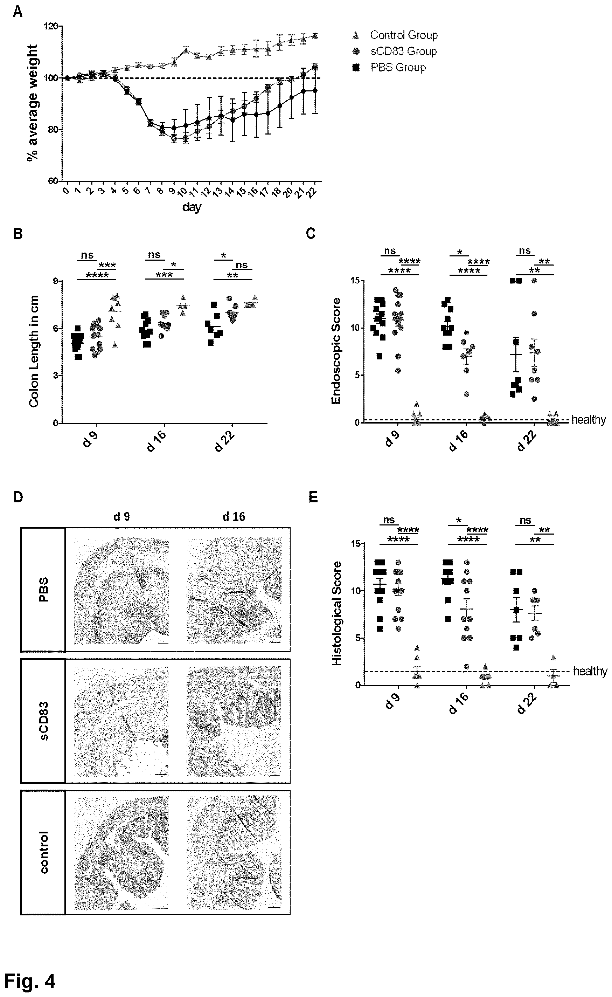

Figure 4 - Intestinal wound healing is accelerated in sCD83-treated mice during the recovery phase of DSS-induced colitis: (A) Average percentage of weight loss over a time period of 22 days. Shown is one representative experiment out of four independent ones. (B) Measurement of the colon length in cm on day 9, 16, and 22. Shown is the Mean ± SEM; PBS group n = 13 / 11 / 8, sCD83 group n = 13 / 9 / 8, control group n = 8 / 4 / 4 mice per group (day 9, 16, 22); pooled data from four independent experiments. Evaluation of colitis severity and intestinal inflammation on day 9, 16 and 22 by (C) high resolution endoscopy; PBS group n = 13 / 8 / 8, sCD83 group n = 13 / 7 / 8, control group n = 8 / 4 / 4 mice per group; Mean ± SEM; (D, E) evaluation of H&E stained colon tissue sections; PBS group n = 13 / 11 / 8, sCD83 group n = 13 / 9 / 8, control group n = 8 / 4 / 4 mice per group; Mean ± SEM. Statistical analysis (B, C, E): Ordinary One-Way ANOVA, Tukey's multiple comparison test; *P < 0.05, **P < 0.01, ***P < 0.001, ****P < 0.0001.

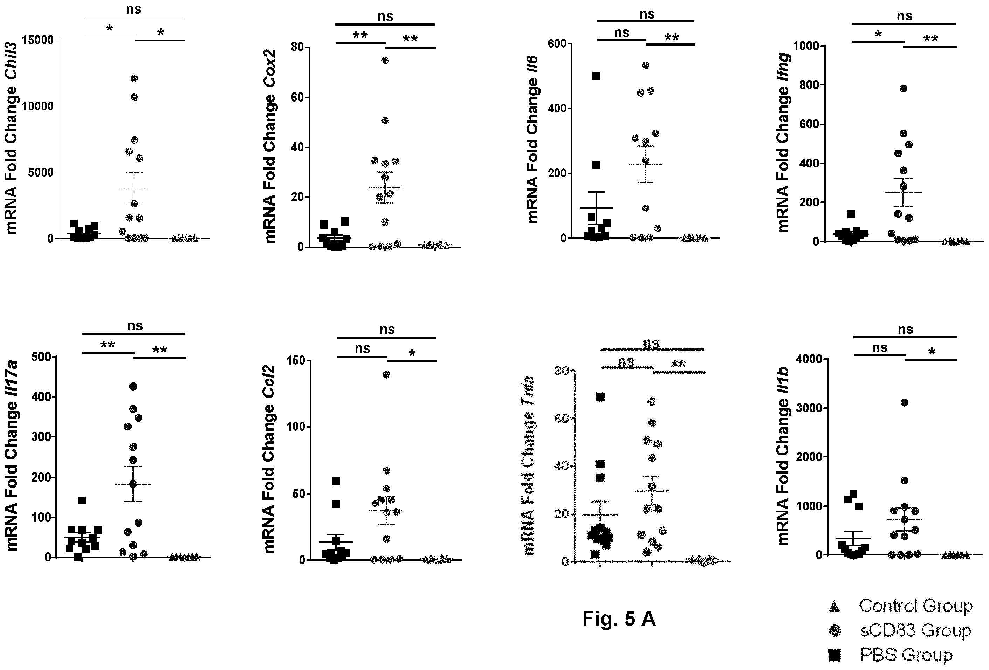

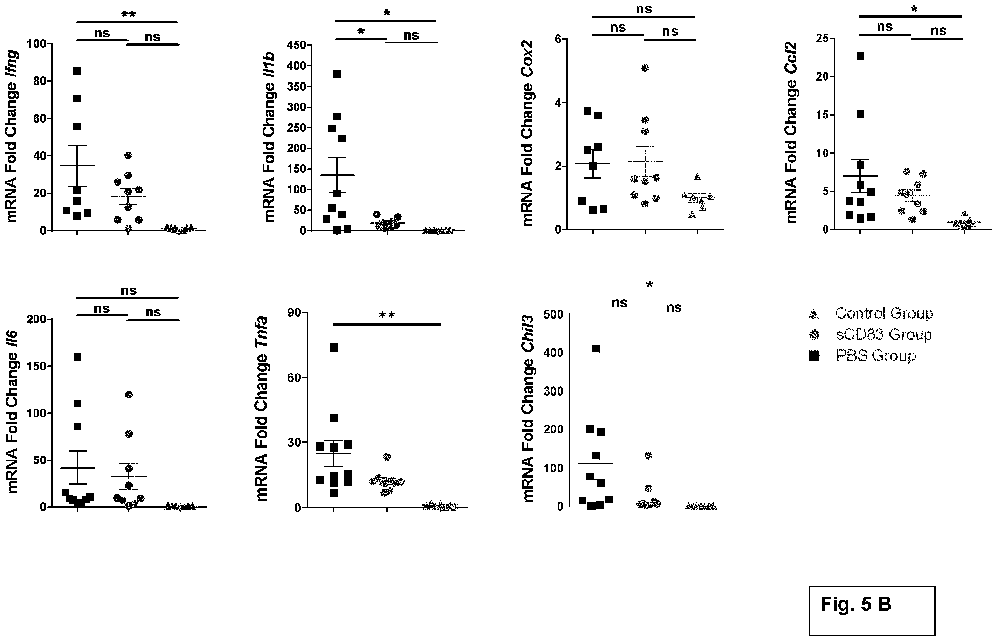

Figure 5 - sCD83 treatment of DSS-challenged mice accelerates their recovery via an enhanced inflammatory response and a faster intestinal wound healing process: RNA was isolated from colon tissue on (A) day 9 and (B) day 16 for the analysis of specific gene transcripts, including *Ifng, Il1b, Il6, Tnfa, Cox2, Ccl2, Chil3, and Il17a* using real-time qPCR. Shown is the Mean ± SEM; PBS group n = 13 / 11, sCD83 group n = 13 / 9, control group n = 8 / 4 mice per group (day 9 / 16); pooled data from four independent experiments; data are normalized to HPRT and relativized to the mean of the control group. Statistical analysis: Ordinary One-Way ANOVA, Tukey's multiple comparison test; significant outliers were determined by the R

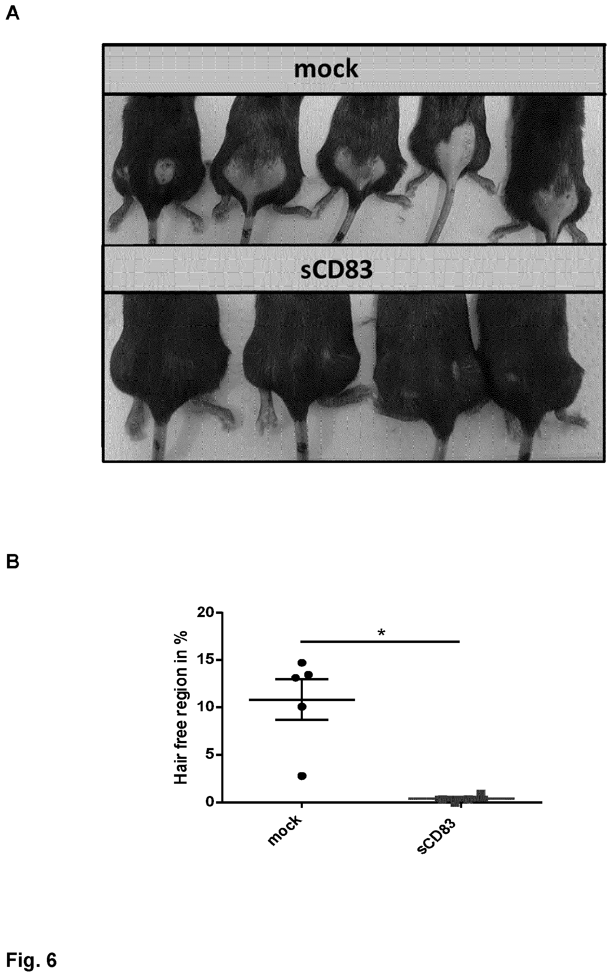

Figure 6 - Hair regrowth is boosted in sCD83-treated mice: (A) Representative pictures from sCD83- (n=4) and mock-treated (n=5) mice. (B) Quantification of bald area relatively to the whole back area of the depicted mice using ImageJ. Data are illustrated as mean ± SEM and statistically analyzed by Mann Whitney. The asterisk represents a statistically significant difference of p < 0.05.OUT method (Q = 0.1%), if necessary.

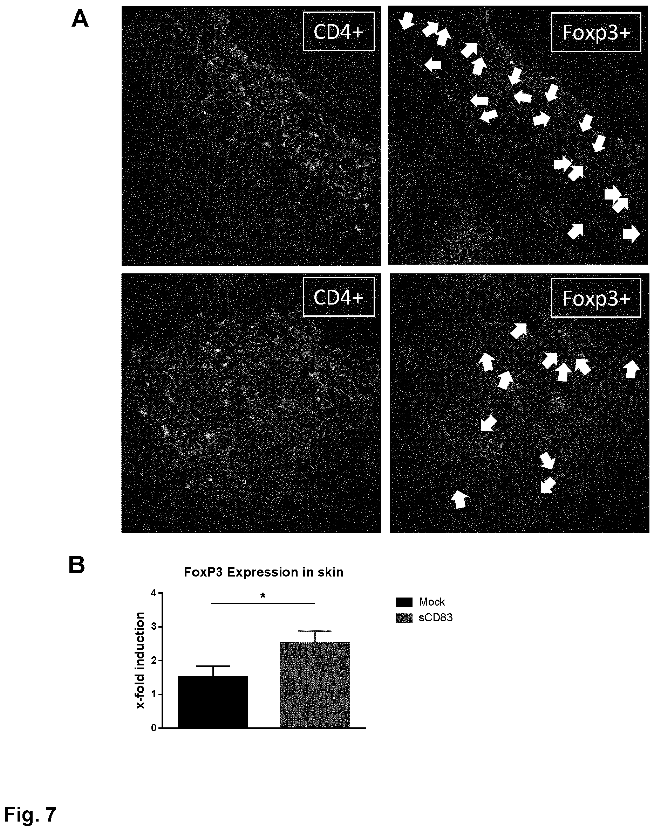

Figure 7 - Tregs play a pivote role in the sCD83-induced hair regrowth: (A) Microscopical analyses using the MELC technique (Eckhardt, J. et al., J Histochem Cytochem 61:125-133 (2013)) to assess CD4⁺/Foxp3⁺ Treg cells and (B) qPCR analyses (sCD83 n=10 and mock n=9), were performed using dorsal skin biopsies, 31 days after depilation. Data are illustrated as mean ± SEM and statistically analyzed using Mann Whitney. Asterisks mark statistically significant difference (*p < 0.05).

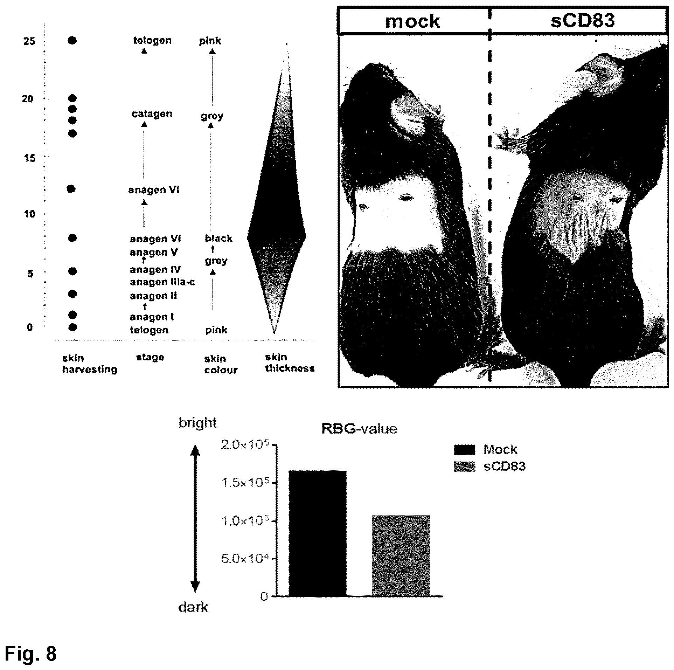

Figure 8 - Hair regrowth was boosted in sCD83-treated mice during cutaneous wound healing: Before the induction of wound lesions, dorsal hair was removed by depilation. Wounds were inflicted using 8 mm skin biopsy punches. Enhanced hair regrowth in sCD83-treated mice is represented by the dark skin color on day 7, as summarized by the hair growth scheme (left-handed). Quantification of skin pigmentation was performed using ImageJ software and the RBG measure plugin. A low RBG-value represents a darker skin tone.

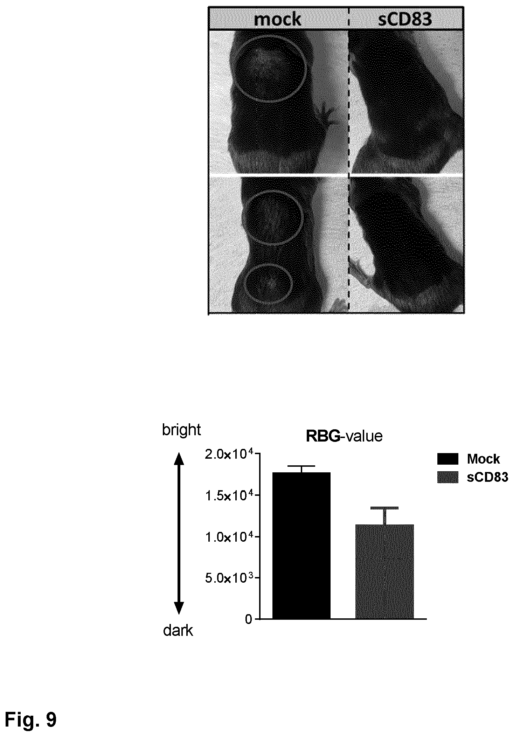

Figure 9 - sCD83 promotes hair growth upon telogen to anagen transition: Dorsal skin hair was depilated in sCD83- and mock-treated mice and images were recorded on day 14. Red circles highlight area of light hair in mock mice, which are not present in the sCD83-group (upper part). Quantification of hair density was performed using ImageJ software and the RBG measure plugin. Dense dorsal hair covers the pale murine skin in sCD83-treated mice, as represented by the lower RBG-value (lower part).

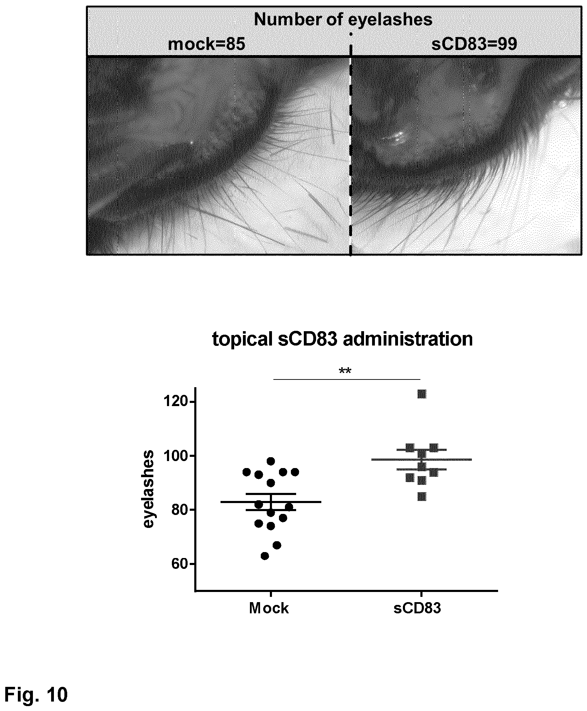

Figure 10 - increases the numbers of eyelashes upon topical administration: In order to assess the effect of sCD83 regarding the growth of eyelashes, 7 µg sCD83 were applied topically thrice a day for a time period of two weeks. Analogous volume of PBS was applied as control. On day 14 eyelids were removed, and the number of eyelashes was determined by two independent investigators (sCD83 n=9 and mock n=14). Data are illustrated as mean ± SEM and statistically analyzed using Mann Whitney. Asterisks mark the statistically significant difference, wherein ** represents a p value of < 0.01.

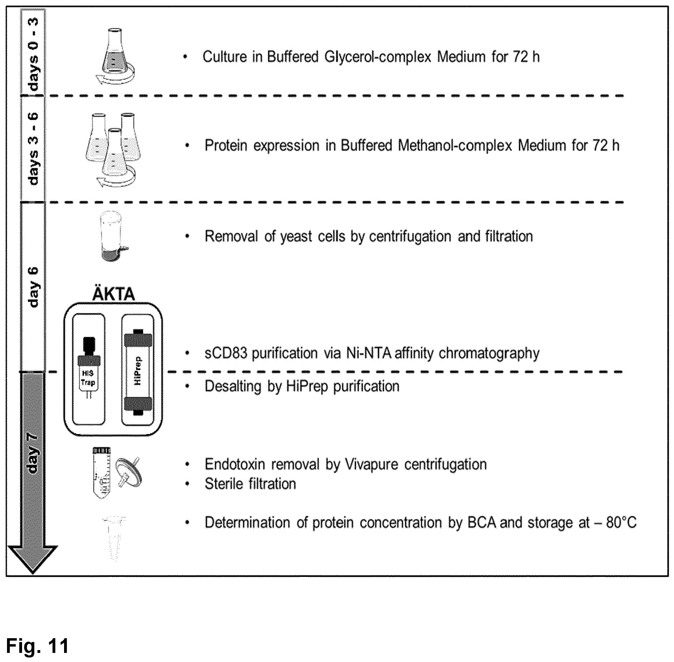

Figure 11 - Flowchart of production and purification of sCD83 from P. pastoris flask cultures: A single colony of N6HIS-sCD83-pPIC9K transformed *P. pastoris* was cultured in buffered Glycerol-complex Medium for 72 h. Thereafter, cells were transferred into buffered Methanol-complex expression-medium for further 72 h. On day 6, culture supernatants were collected by centrifugation and filtration for subsequent His-tagged sCD83-purification via Ni-NTA affinity chromatography. sCD83 was eluted in 12 ml elution buffer and stored o.n. at 4 °C. The next day, desalting procedure was performed by HiPrep purification and endotoxin contaminations were removed by vivapure systems. Finally, the protein was sterile filtered and protein yields were determined by BCA method. sCD83 aliqiots were stored at - 80 °C.

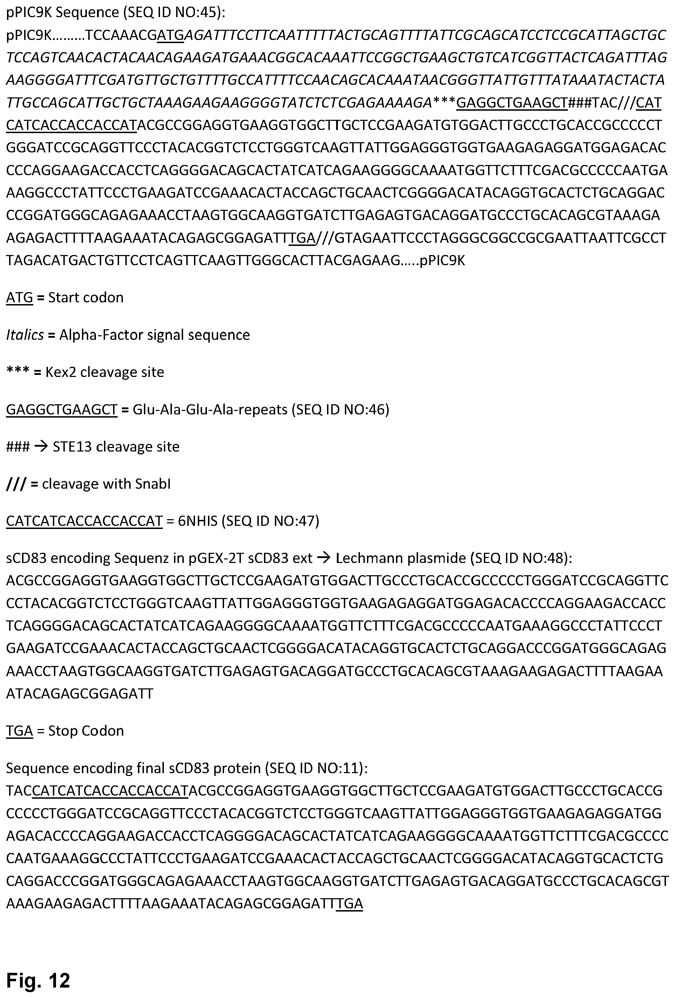

Figure 12 - pPIC9K-expression vector: Functional sequences and elements of the pPIC9K-expression vector used in Example 3.

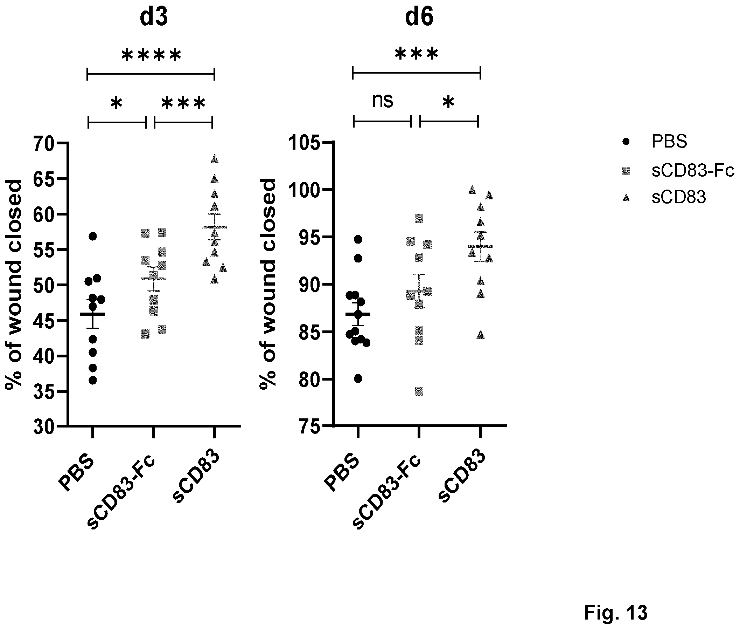

Figure 13 - Progress of cutaneous wound healing is significantly enhanced in systemically sCD83-treated mice compared to sCD83-Fc fusion protein treated mice. Results of Example 4: Mice were systemically treated using a daily dose of 100 µg sCD83 fused to the N-terminus of the Fc region of human IgG1 (termed sCD83-Fc; Creative Biomart - CD83-150H; (square; n=10), sCD83 (triangle; n=10) or saline (bullet; n=12). Wound area was measured on day 0, 3 and 6 using a caliper and wound closure was determined for day 3 and 6 relatively to the day 0 value. Data are shown as mean with single values ± SEM. Statistical significance was evaluated using two-way ANOVA. Asterisks mark statistically significant difference (*p < 0.05, **p < 0.01, ***p < 0.001, and ****p < 0.0001) while "ns" indicates that there is no statistical significance.

### Detailled Description of the Invention

According to aspect (1) of the invention a soluble form of a member of the CD83 family of proteins is used for wound healing. The CD83 protein is a sCD83 protein as disclosed in the WO 04/046182 referred to above, namely a sCD83 protein that consists of amino acid residues 20 to 144 of SEQ ID NO: 2, including derivatives of the sCD83 proteins having up to 10 amino acids attached to the C- or N-terminus of the sCD83 protein and/or proteins having up to 10 amino acids, preferably one to five amino acids, of the sCD83 substituted. In particular, such sCD83 proteins include sCD83 proteins having additional sequences attached to its C- or N-terminus, e.g. those carrying part of a transmembrane domain at their C-terminus or carrying at their N-terminus a short functional peptide (Gly-Ser-Pro-Gly) may be used.

In a similar manner, nucleic acids or vectors coding for these sCD83 proteins may be used for wound healing (not part of the present invention). In particular, DNA sequences comprising nucleotides 58 to 432, more preferably 58 to 435 of SEQ ID NO: 1 may be used.

The term "soluble form" of the CD83 family of proteins is used here to define a proteinaceous molecule that has at least a portion of the extracellular domain of a member of the CD83 family of proteins but does not have an amino acid sequence that is capable of anchoring said molecule to the membrane of a cell in which it is expressed. The nucleic acid sequence encoding human CD83 protein as well as the amino acid sequence of CD83 are described in Zhou, L.J. et al. (1992) J. Immunol. 149(2):735-742 (Genbank accession number Z11697) and are provided in SEQ ID NO:1 and SEQ ID NO:2, respectively.

Aside from the human sCD83 with amino acid residues 20 to 144 of SEQ ID NO:2, the sCD83 may be derived from other members of the CD83 family of proteins, provided they differ in 10 amino acids or less from the human CD83 with amino acid residues 20 to 144 of SEQ ID NO:2. Such CD83 include the mouse HB15 protein that is encoded by the nucleic acid sequence of SEQ ID NO:3 and is represented by the amino acid sequence provided in SEQ ID NO:4, (Genbank accession number NM_009856 (Berchthold et al.)).

Other naturally occurring members of the CD83 family of proteins can be obtained by hybridizing a nucleic acid comprising, for example, all or the extracellular portion of the human CD83 coding region or mouse HB15 coding region to various sources of nucleic acids (genomic DNA, cDNA, RNA) from other animals, preferably mammals, or from other tissues of the same organism.

Hybridization refers to the binding between complementary nucleic acid sequences (e.g., sense/antisense, siRNA). As is known to those skilled in the art, the Tₘ (melting temperature) refers to the temperature at which the binding between sequences is no longer stable. As used herein, the term "selective hybridization" refers to hybridization under moderately stringent or highly stringent conditions, which can distinguish CD83 related nucleotide sequences from unrelated sequences.

In nucleic acid hybridization reactions, the conditions used in order to achieve a particular level of stringency will vary, depending on the nature of the nucleic acids being hybridized. For example, the length, degree of sequence complementarity, sequence composition (e.g., the GC v. AT content), and type (e.g., RNA v. DNA) of the hybridizing regions can be considered in selecting particular hybridization conditions. An additional consideration is whether one of the nucleic acids is immobilized, for example, on a filter.

In general, the stability of a nucleic acid hybrid decreases as the sodium ion decreases and the temperature of the hybridization reaction increases. An example of moderate stringency hybridization reaction is as follows: 2 x SSC/0.1 SDS at about 37 °C or 42 °C (hybridization conditions); 0.5 x SSC/0.1% SDS at about room temperature (low stringency wash conditions); 0.5 x SSC/0.1 % SDS at about 42 °C (moderate stringency wash conditions). An example of high stringency hybridization conditions is as follows: 2 x SSC/0.1% SDS at about room temperature (hybridization conditions); 0.5 x SSC/0.1% SDS at about room temperature (low stringency wash conditions); 0.5 x SSC/0.1% SDS at about 42 °C (moderate stringency wash conditions); and 0.1 x SSC/0.1% SDS at about 65 °C (high stringency conditions).

Typically, the wash conditions are adjusted so as to attain the desired degree of stringency. Thus, hybridization stringency can be determined, for example, by washing at a particular condition, e.g., at low stringency conditions or high stringency conditions, or by using each of the conditions, e.g., for 10-15 min each, in the order listed above, repeating any or all of the steps listed. Optimal conditions for selective hybridization will vary depending on the particular hybridization reaction involved, and can be determined empirically.

Once a nucleic acid encoding a naturally occurring CD83 protein has been cloned, the extracellular domain can be determined by comparison of the extracellular domain of known CD83 molecules with that of the cloned CD83 sequence. A soluble form of a given naturally occurring CD83 protein can then be expressed recombinantly using the techniques as described herein. For example, a nucleic acid encoding a soluble form of CD83 can be produced, inserted into a vector and transformed into prokaryotic or eukaryotic host cells using well known techniques described herein and further known in the art (Sambrook et al. Molecular Cloning: A Laboratory Manual, Cold Spring Harbor Laboratory, N.Y.,1989).

Thus, when cloning in bacterial systems, constitutive promoters such as T7 and the like, as well as inducible promoters such as pi, of bacteriophage X, plac, ptrp, ptac (ptrp-lac hybrid promoter) may be used. When cloning in mammalian cell systems, constitutive promoters such as SV40, RSV, CMV including CMV-IE, and the like or inducible promoters derived from the genome of mammalian cells (e.g., metallothionein promoter) or from mammalian viruses (e.g., the mouse mammary tumor virus long terminal repeat; the adenovirus late promoter) may be used. Promoters produced by recombinant DNA or synthetic techniques may also be used to provide for transcription of the nucleic acid sequences of the invention.

Mammalian expression systems which utilize recombinant viruses or viral elements to direct expression may be engineered. For example, when using adenovirus expression vectors, nucleic acid of interest may be ligated to an adenovirus transcription/translation control complex, e.g., the late promoter and tripartite leader sequence. Alternatively, the vaccinia virus 7.5K promoter may be used.

Of particular interest are vectors based on bovine papilloma virus (BPV) which have the ability to replicate, as extrachromosomal elements. Shortly after entry of an extrachromosomal vector into mouse cells, the vector replicates to about 100 to 200 copies per cell. Because transcription of the inserted cDNA does not require integration of the plasmid into the host's chromosome, a high level of expression occurs. These vectors can be used for stable expression by including a selectable marker in the plasmid, such as the neo gene, for example. Alternatively, the retroviral genome can be modified for use as a vector capable of introducing and directing the expression of the nucleic acid of interest in host cells. High level expression may also be achieved using inducible promoters, including, but not limited to, the metallothionein RA promoter and heat shock promoters.

In yeast, a number of vectors containing constitutive or inducible promoters, e.g. pPIC9K, may be used. A constitutive yeast promoter such as ADH or LEU2 or an inducible promoter such as GAL may be used. Alternatively, vectors that facilitate integration of foreign nucleic acid sequences into a yeast chromosome, via homologous recombination for example, are known in the art and can be used.

A nucleic acid of interest encoding a soluble form of a member of the CD83 family of proteins for use according to the present invention may be inserted into an expression vector for expression in vitro (e.g., using *in vitro* transcription/ translation assays or commercially available kits), or may be inserted into an expression vector that contains a promoter sequence which facilitates transcription and/or translation in either prokaryotes or eukaryotes (e.g., an insect cell) by transfer of an appropriate nucleic acid into a suitable cell. A cell into which a vector can be propagated and its nucleic acid transcribed, or encoded polypeptide expressed, is referred to herein as a "host cell".

The term also includes any progeny of the subject host cell. Moreover, a nucleic acid of interest according to the present invention may be inserted into an expression vector for expression in vivo for somatic gene therapy. With these vectors, for example, retroviral vectors, Adenovirus vectors, Adeno-associated virus vectors, plasmid expression vectors, the nucleic acids of the invention are expressed upon infection/introduction of the vector into specific cells such as immune cells, stem cells, endothelial cells, epithelial cells, fibroblasts as well as keratinocytes and others.

Host cells include microorganisms such as bacteria, yeast, insect and mammalian organisms. For example, bacteria transformed with recombinant bacteriophage nucleic acid, plasmid nucleic acid or cosmid nucleic acid expression vectors containing a nucleic acid of interest; yeast transformed with recombinant yeast expression vectors containing a nucleic acid of interest; plant cell systems infected with recombinant virus expression vectors (e.g., cauliflower mosaic virus, CaMV; tobacco mosaic virus, TMV) or transformed with recombinant plasmid expression vectors (e.g., Ti plasmid) containing a nucleic acid of interest; insect cell systems infected with recombinant virus expression vectors (e.g., baculovirus) containing a nucleic acid of interest; or animal cell systems infected with recombinant virus expression vectors (e.g., retroviruses, adenovirus, vaccinia virus) containing a nucleic acid of interest, or transformed animal cell systems engineered for stable expression.

For long-term expression of the soluble forms of members of the CD83 family of proteins in host cells, stable expression is preferred. Thus, using expression vectors which contain viral origins of replication, for example, cells can be transformed with a nucleic acid of interest controlled by appropriate control elements (e.g., promoter/enhancer sequences, transcription terminators, polyadenylation sites). Optionally, the expression vector also can contain a nucleic acid encoding a selectable or identifiable marker conferring resistance to a selective pressure thereby allowing cells having the vector to be identified, grown and expanded. Alternatively, the selectable marker can be on a second vector that is cotransfected into a host cell with a first vector containing an invention polynucleotide.

A number of selection systems may be used, including, but not limited to the herpes simplex virus thymidine kinase gene, hypoxanthine-guanine phosphoribosyltransferase gene, and the adenine phosphoribosyltransferase genes can be employed in tk-, hgprt or aprt cells respectively. Additionally, antimetabolite resistance can be used as the basis of selection for dhfr, which confers resistance to methotrexate; the gpt gene, which confers resistance to mycophenolic acid; the neomycin gene, which confers resistance to the aminoglycoside G-418; and the hygromycin gene, which confers resistance to hygromycin. Additional selectable genes have been described, namely trpB, which allows cells to utilize indole in place of tryptophan; hisD, which allows cells to utilize histinol in place of histidine; and ODC (ornithine decarboxylase) which confers resistance to the omithine decarboxylase inhibitor, 2-(difluoromethyl)-DL-onithine, DFMO.

As used herein, the term "transformation" means a genetic change in a cell following incorporation of DNA exogenous to the cell. Thus, a "transformed cell" is a cell into which (or a progeny of which) a DNA molecule has been introduced by means of recombinant DNA techniques.

Transformation of a host cell with DNA may be carried out by conventional techniques known to those skilled in the art. For example, when the host cell is a eukaryote, methods of DNA transformation include, for example, calcium phosphate co-precipitates, conventional mechanical procedures such as microinjection, electroporation, insertion of a plasmid encased in liposomes, and viral vectors. Eukaryotic cells also can be cotransformed with DNA sequences encoding a nucleic acid of interest, and a second foreign DNA molecule encoding a selectable phenotype, such as those described herein. Another method is to use a eukaryotic viral vector, such as simian virus 40 (SV40) or bovine papilloma virus, to transiently infect or transform eukaryotic cells and express the protein.

Following transformation, the soluble form of CD83 may be isolated and purified in accordance with conventional methods. For example, lysate prepared from an expression host (e.g., bacteria) can be purified using HPLC, size-exclusion chromatography, gel electrophoresis, affinity chromatography, or other purification technique. Substantially pure proteins can also be obtained by chemical synthesis using a peptide synthesizer (e.g. Applied Biosystems, Inc., Foster City, CA; Model 430A or the like).

Furthermore, the sCD83 protein for use in the aspect (1) of the present invention may be a dimeric or trimeric structure of the soluble form of CD83 as described in WO04/046182 and Heilingloh, C.S. et al., JMB J Mol Biol. 429(8):1227-1243 (2017), respectively. Preferably the dimeric or trimeric structure is a homodimer/- trimer. Dimerisation/trimerisation may be achieved through formation of one or more disulfide bonds between the cysteine residues present within the monomeric form of the soluble CD83 protein (which are present at aa 12, 27, 35, 100, 107, 129, 163 in SEQ ID NO:2), or by means of a bifunctional linker molecule (e.g. a diamine, a dicarboxylic acid compound or the like) connecting same or different functional moieties (e.g. carboxy groups, amino groups, hydroxy groups, mercapto groups) within the monomeric form of the soluble CD83 protein. The latter also includes the use of polypeptide linkers (e.g. out of small polar amino acid residues such as -[(Gly)ₓSer]_{y}- (where x is e.g. 3 or 4 and y is e.g. 1 to 5)) to yield dimeric or trimeric structures which can directly be produced by recombinant techniques. Particularly preferred is a homodimer (such as a homodimer comprising amino acid residues 20 to 144 of SEQ ID NO:2 or 1 to 130 of SEQ ID NO:8) connected via a disulfide bond between the fifth cysteine residue of the soluble CD83 (i. e., the cysteine residue corresponding to aa 129 in SEQ ID NO:2 and aa 114 in SEQ ID NO:8).

The sCD83 protein for use in the present invention also include derivatives of soluble forms of members of the CD83 family of proteins according to the invention as mentioned above in which one or more amino acids has been added, deleted, substituted, inserted or inverted as long as these derivatives remain soluble as defined above and are capable of exhibiting a healing function. It also includes splice variants of the CD83 compounds mentioned hereinbefore.

Particular preferred additions are those where the soluble CD83 protein as defined hereinbefore has one or more amino acid residues derived from the neighboring intracellular domain at its C-terminus, preferably the soluble CD83 protein comprises amino acid residues 20 to 145 of SEQ ID NO:2; or has functional sequences attached to its N-terminus, preferably functional sequences of up to 10 amino acid residues, and most preferably carries at the N-terminus the additional amino acids Gly-Ser-Pro-Gly, or 6HIS.

When one or more amino acids of a soluble form of a member of the CD83 family of proteins is substituted, it is preferred that the one or more amino acids are conservatively substituted. For example, conservative substitutions include substitutions in which aliphatic amino acid residues such as Met, Ile, Val, Leu or Ala are substituted for one other. Likewise, polar amino acid residues can be substituted for each other such as Lys and Arg, Glu and Asp or Gln and Asn.

Particular substitution muteins of the sCD83 protein for use in the invention are those, where the sCD83 protein is a monomer CD83 protein where one or more of the cysteine residues have been substituted by same or different short and/or polar amino acid residue(s) as described in WO04/046182. Preferably the small and/or polar amino acid residues are selected from serine, alanine, glycine, valine, threonine, preferably is serine. Moreover, it is preferred that one cysteine residue, more preferably the fifth cysteine residue, has been replaced. Most preferably the soluble CD83 protein comprises amino acid residues 20 to 144 of SEQ ID NO:2, where the cysteine residue at position 129 has been replaced by a serine residue, or amino acid residues 1 to 130 of SEQ ID NO:10. Such defined monomeric molecules possess particular importance for pharmaceutical application.

According to the invention, derivatives of a CD83 protein also include derivatives in which one or more of the amino acids therein has an altered side chain. Such derivatized polypeptides include, for example, those comprising amino acids in which free amino groups form amine hydrochlorides, p-toluene sulfonyl groups, carobenzoxy groups; the free carboxy groups form salts, methyl and ethyl esters; free hydroxl groups that form 0-acyl or 0-alkyl derivatives as well as naturally occurring amino acid derivatives, for example, 4-hydroxyproline, for proline, 5-hydroxylysine for lysine, homoserine for serine, omithine for lysine. Also included are amino acid derivatives that can alter covalent bonding, for example, the disulfide linkage that forms between two cysteine residues that produces a cyclized polypeptide.

An sCD83 protein or derivative thereof can have a native glycosylation pattern of a CD83 molecule or an altered glycosylation pattern or can be non-glycosylated as long as these molecules are soluble as defined above and are capable of exhibiting a wound healing function and/or promote hair regrowth.

In a preferred embodiment, tThe sCD83 for use in the present invention consists of amino acids 20 to amino acids 144, more preferably amino acids 20 to 145, of the human CD83 protein as depicted in SEQ ID NO:2 or amino acids 1 to 130 of SEQ ID NO:8.

The sCD83 for use in the present invention may consist of amino acids 22 to 135 of the mouse HB15 protein as depicted in SEQ ID NO:4.

The sCD83 for use in the present invention may be a human sCD83 that consistsof amino acids 8 to 133 of the N6HIS-sCD83 protein as prepared according to Example 3 below and as depicted in SEQ ID NO:12.

The nucleic acids for use in wound healing (not part of the present invention) can be in the form of DNA (deoxyribonucleic acid) which contains the bases adenine, thymine, guanine and cytosine or RNA (ribonucleic acid) which contains the bases adenine, uracil, guanine and cytosine or mixtures of the two.

When the nucleic acid molecule for use in wound healing is derived from human CD83 protein, the portion of the coding region is preferably from nucleotide 58 to 432 of the sequence in SEQ ID NO:1. Alternatively, the portion of the coding region is from nucleotide 58 to 435 of the sequence in SEQ ID NO:1.

When the nucleic acid molecule for use in the invention is derived from the mouse HB15 protein, the portion of the coding region is preferably from about nucleotide 76 to 418 of the sequence in SEQ ID NO:3.

A nucleic acid that encodes a protein for use according to the invention may be inserted into a vector. The term "vector" refers to a plasmid, virus or other vehicle known in the art that can be manipulated by insertion or incorporation of a polynucleotide. Such vectors can be used for genetic manipulation (i.e., "cloning vectors") or can be used to transcribe or translate the inserted polynucleotide ("expression vectors"). A vector generally contains at least an origin of replication for propagation in a cell and a promoter. Control elements, including expression control elements as set forth herein, present within an expression vector are included to facilitate proper transcription and translation (e.g., splicing signal for introns, maintenance of the correct reading frame of the gene to permit in-frame translation of mRNA and, stop codons). The term "control element" is intended to include, at a minimum, one or more components whose presence can influence expression, and can also include additional components, for example, leader sequences and fusion partner sequences.

The sCD83 protein for use in wound healing of aspect (1) of the invention also includes a "medical composition" with the sCD3 for use in wound healing. Such medical composition may comprise - apart from the sCD83 protein - pharmaceutically acceptable salts, buffers, carriers, excipients. It may also comprise one or more additional pharmaceutically active compounds.

The sCD83 protein or the medical composition with said sCD83 for use in wound healing of aspect (1) of the invention comprises, but is not limited to, systemic administration of the sCD83 for accelerated wound closure of cutaneous wounds, including hard-to-heal wounds of elderly-, diabetic-, or patients under immunosuppressant or corticosteroidal medication. It also comprises topical administration, preferably in form of a gel-based sCD83 solution, for wound closure of skin lesions, including locally restricted wounds, chronic wounds (hard-to-heal wounds of elderly-, diabetic-, or patients under immunosuppressant or corticosteroidal medication) and transplantation induced wounds.

The discussion provided hereinbefore equally applies to the sCD83 protein, dimeric or trimeric form, and/or functional derivative thereof, and the composition comprising such sCD83 protein, for use in therapeutic hair growth of aspects (2) and (3) of the invention. The therapeutic hair growth comprises systemic or topical sCD83 applications after physical stress/medication, disease and/or depilation. Any of the sCD83 proteins as defined above may be used for the topical and systemic applications, for systemic applications. The "composition" for use in therapeutic hair growth may comprise - apart from the sCD83 protein, or derivative thereof, or nucleic acids coding therefore - pharmaceutically acceptable salts, buffers, carriers, excipients. It may also comprise one or more additional pharmaceutically active compounds.

The discussion provided hereinbefore equally applies to the cosmetic non-therapeutic method for the acceleration of hair growth and for hair care of aspect (4) of the invention, which comprises systemically or topically applying a composition comprising the sCD83 protein to the human or animal skin or hair. The "composition" utilized in the method of aspect (45) may comprise - apart from the sCD83 protein, or derivative thereof - cosmetically/dermatologically or pharmaceutically acceptable salts, buffers, carriers, and excipients., respectively. The invention is further explained by the following examples.

### Examples

### Materials and Methods

Mice: Female C57BL/6 mice (8-10 weeks old) were purchased from Charles River Laboratories (Sulzfeld) and maintained under pathogen free conditions according to the institutional and national guidelines for the care and use of laboratory animals.

Experimental design of the wound healing model: At day 0, 8 to 10 weeks old female CD57BL/6 mice were anesthetized and wounds were induced using 6 mm punches (pfm medicals). These skin samples were used as day 0 control for subsequent analyses. Two treatment groups were included into these in vivo experiments, namely: (I) sCD83 protein (II) sCD83-Fc fusion protein. A daily dose of 100 µg sCD83, 100 µg sCD83 fused to the N-terminus of the Fc region of human IgG1 (termed sCD83-Fc; Creative Biomart - CD83-150H) or the corresponding volume of PBS was administered i.p. from day 0 until day 7. Before scoring, the eschar was removed and wound area was determined by caliper (length and width). Biopsies were obtained as 8 mm punches (pfm medicals) around the former wound area on day 3, 6, 7 and 12, respectively and samples were either fixed in 4% paraformaldehyde (PFA) for histological analyses, snap frozen in liquid nitrogen for protein extraction or stored in RNAlater (Thermo Fisher Scientific) at -80 °C for subsequent RNA-analyses. For topical sCD83-application, dorsal hair was removed by depilation to ensure a uniform deposition of the hydrogel-stabilized sCD83 protein. On this purpose, mice were anesthetized, the eschar was removed and 50 µg sCD83-hydrogel were applied on a daily base.

Experimental design for the DSS-induced colitis model: Female C57BL/6 mice, at an age of 8 to 10 weeks, were randomly divided into three groups: control-, PBS-, and sCD83 group. Each group was subdivided into a maximum of three more groups representing the different time points of analyses, i.e. day 9, day 16 and day 22. Symptoms were induced by giving tap water containing 3% DSS for 5 d. Additionally in the sCD83 groups, each mouse received an intraperitoneal (i.p.) injection of 100 µg sCD83/d for 6 d. Accordingly, in the PBS groups mice received i.p. injections of sterile 100 µl PBS/d. Clinical monitoring included the daily measurement of body weight, after reaching the different time points of analyses, a high-resolution video endoscopic monitoring of the colitis activity in the anesthetized mice, and measurement of the colon length after veterinary euthanasia of the animals. Animals were dissected to use the mLNs and the colon for further analyses. Therefor an approximately 1 cm section of the distal colonic part, was fixed in 4% PFA for hematoxylin and eosin (HE) staining, and was assessed under a light microscope. For the analysis of inflammatory and anti-inflammatory parameters on mRNA level, a small piece of the ensuing colon segment as well as mLNs were snap frozen in RNALater solution using liquid nitrogen, and stored at -80 °C.

Evaluation of endoscopic score: Colitis severity was assessed using a miniature endoscope for high-resolution colon monitoring, as described before (Becker, C. et al., Nat Protoc 1:2900-2904 (2006); Becker, C. et al., Gut 54:950-954 (2005)) and the help of Dr. E. Martini (Department of Medicine 1, University Hospital Erlangen). Mice were analyzed regarding their stool consistency, translucency of the colon wall, vascularity, granularity, and fibrin deposit. The overall endoscopic severity score ranged from a minimum of 0 to a maximum of 15 and was determined by adding up the singly scores of the individual parameters, each ranking from 0 to 3. Control animals with an overall endoscopic score ranging between 0 and 3 were considered as healthy.

Preparation of Hydrogel: sCD83 hydrogels were prepared by the addition of 4% (w/v) hydroxyethylcellulose (Natrosol^{®} 250 HX Pharm; Caelo) to sCD83 solutions. The hydroxyethylcellulose was dispersed on the surface of the aqueous SD83 solutions and allowed to gel overnight. The mock (PBS) samples were prepared identically. In addition, chemical penetration enhancers (CPE) could also be used. Cell extraction from skin biopsies: Biopsies of 8 mm were isolated on day 3 and 6, cut into small pieces using a surgical scalpel and transferred into a 1.5 ml tube containing 1 ml DMEM/F12 (Thermo Fisher Scientific), and mixed with Liberase TL (Sigma Aldrich) in a final concentration of 0.26 WU/ml. The samples wereincubated for 30 min at 37 °C and 700 rpm on a shaking incubator. Afterwards, the suspension was filtered through a 70 µm cell strainer using the back-site of a 5 ml syringe plunger. The strainer was flushed with 5 ml DMEM/F12 and the flow through was centrifuged for 5 min (300 g at 4 °C). After an additional washing step with 5 ml PBS (5 min, 300 g at 4 °C), the pellet was resuspended in 1 ml PBS, filtered through a 35 µm cell strainer and used for subsequent surface staining for flow cytometry.

Protein isolation from skin tissue: Proteins were isolated from murine skin biopsies, which were cut by a surgical scalpel into small pieces. Next, the skin tissue was transferred into innuSPEED lysis tubes W (Analytic Jena), treated with 40 mg/ml lysis buffer (10% glycerin, 2 mM EDTA, 137 mM NaCl, 50 mM Tris, 0.5% NP40, PMSF, NaF1M, Na₃VO₄) and shredded using the SpeedMill PLUS (Analytic Jena), until the tissue was homogenized. Afterwards, lysates were centrifuged (16.000 g, 3 min, 4 °C) and transferred into 1.5 ml Eppendorf tubes. Upon a second centrifugation step (16.000 g, 20 min, 4 °C), supernatants were collected, and protein concentration was determined using BCA according to the manufacturer's instructions (Thermo Fisher Scientific).

Western blot analyses: Protein lysates were denatured in reducing Laemmli buffer (95°C, 5min), separated via 10 % SDS PAGE and blotted onto a nitrocellulose membrane (GE Healthcare). The membrane was blocked in 1x Rotiblock (Carl Roth) and subsequently probed with the following primary antibodies: rabbit-anti-Vimentin (Clone D21H3, Cell Signaling) and mouse-anti-β-Aktin (Clone AC-74, Sigma), diluted 1:1000 in 1x Rotiblock and incubated overnight at 4 °C. Afterwards, membranes were washed and the appropriate HRP-labelled secondary antibody (diluted 1:5000 in 1x Rotiblock), was added. Signals were detected using the ECL Prime Detection reagent (GE Healthcare) and digitally quantified using the Image J software.

Flow cytometry: Cell samples from skin biopsies were centrifuged for 3 min (500 g at 4 °C) and the cell pellet was resuspended in 50 µl PBS (supplemented with 1 mM EDTA), containing fluorescence-coupled antibodies against CD45 (PerCP (clone 30-F11)), CD3 (BV421(clone 17A2), CD11b (PE-Cy7 (clone M1/70), B220 (PE (clone RA3-6B2), F4/80 (FITC (clone BM8), Ly6C (APC-Cy7 (clone HK1.4), Ly6G (APC (clone 1A8) and LIVE/DEAD Fixable Aqua DEAD Cell stain (Thermo Fisher Scientific). All antibodies (except for B220, from BD) were purchased from BioLegend. After incubation for 30 min at 4 °C, cells were washed with 500 µl PBS (500 g, 3 min at 4 °C), resuspended in 100 µl PBS and assessed using a FACSCantoII flow cytometer (BD).

RNA extraction, cDNA synthesis and qPCR analyses (for Example 1): Biopsies were collected and stored in RNA later (Thermo Fisher Scientific) at -80 °C. For RNA isolation the samples were thawed gently on ice and homogenization was performed in 400 µl RLT buffer (supplemented with 1% β-mercaptoethanol), using innuSPEED Lysis Tube I and SpeedMill PLUS (both from AnalytikJena). After three homogenization steps, each 20 s with 5 min cooldown at -20 °C, the suspension was centrifuged for 3 min at 16.000 g and the supernatant was collected for RNA extraction using the RNeasy Plus Mini Kit (Qiagen), according to manufacturer's protocol. cDNA synthesis was performed according to the First Strand cDNA Synthesis Kit manual (Thermo Fisher). Ribosomal protein L4 was used as housekeeping gene. The reaction and quantification were performed using the CFX96 Touch (Bio-Rad). Nucleotide sequences of the murine primer pairs are provided in the following table.

| Name | Forward (SEQ ID NO) | Reverse (SEQ ID NO) |
|---|---|---|
| IL-17A | TCCAGAAGGCCCTCAGACTA (13) | AGCATCTTCTCGACCCTGAA (14) |
| FoxP3 | CCCAGGAAAGACAGCAACCTT (15) | CCTTGCCTTTCTCATCCAGGA (16) |
| RANKL | CAGCCATTTGCACACCTCAC (17) | CCCGATGTTTCATGATGCCG (18) |
| TNF-α | GTGATCGGTCCCCAAAGGG (19) | CCAGCTGCTCCTCCACTTG (20) |
| IL-1β | TGCCACCTTTTGACAGTGATG (21) | ATGTGCTGCTGCGAGATTTG (22) |
| Rpl4 | GCTGAACCCTTACGCCAAGA (23) | TCTCGGATTTGGTTGCCAGT (24) |
| IFN-γ | GCTTTGCAGCTCTTCCTCAT (25) | GTCACCATCCTTTTGCCAGT (26) |
| IL-10 | CCAAGCCTTATCGGAAATGA (27) | TTTTCACAGGGGAGAAATCG (28) |
| TGFβ | TGGAGCAACATGTGGAACTCTA (29) | AGACAGCCACTCAGGCGTATC (30) |
| IFN-γ | GCTTTGCAGCTCTTCCTCAT (31) | GTCACCATCCTTTTGCCAGT (32) |
| IL-6 | ACAAAGCCAGAGTCCTTCAGAG (33) | GAGCATTGGAAATTGGGGTAGG (34) |
| Cox2 | GCTGTACAAGCAGTGGCAAA(35) | CCCCAAAGATAGCATCTGGA(36) |
| CCL2 | GAGAGCTACAAGAGGATCACC(37) | GATCTCATTTGGTTCCGATCC (38) |
| Chil3 | GACTTGCGTGACTATGAAGC (39) | TGAATATCTGACGGTTCTGAGG (40) |
| VEGF | ACATTGGCTCACTTCCAGAAACA (41) | GGTTGGAACCGGCATCTTTATC (42) |
| SLPI | GGCCTTTTACCTTTCACGGTG (43) | TACGGCATTGTGGCTTCTCAA (44) |

Histology: For preparation of skin and colon tissue samples for histological analyses, skin biopsies were obtained as 8 mm punches (pfm medicals) around the former wound area on day 3, 6 and 7, respectively and samples were fixed in 4% paraformaldehyde (PFA) for histological analyses. Small colon samples were prepared by cutting approximately 0.5 cm of the distal part of the colon, then fixed in 4 % PFA at room temperature overnight, and washed in PBS. Afterwards, they underwent a dehydration process via increasing alcohol concentrations and a last acetone incubation step. Next, the colonic samples were embedded in paraffin and tissue sections were prepared by cutting 1 µm to 5 µm thick slices with the microtome, layed onto coated slides, dried and stored at RT for later staining procedures.

Histological staining procedure for tissue structures of the colon and skin samples using Mayer's hematoxylin and eosin (H&E) was performed as follows:
For the complete removal of paraffin, the sections were initially incubated at 60 °C overnight and afterwards washed in xylol (2 times for 2-5 min). Then, the sections underwent a rehydration process through decreasing alcohol concentrations and a final washing step. For the staining of basophilic tissue structures, the sections were incubated in freshly filtered hematoxylin, followed by the incubation in eosin-g-solution (1 g eosin in 100 ml VE-water and 1 droplet of glacial acetic acid), to stain eosinophilic tissue structures. After each staining step, the sections were rinsed in running tap water, to remove excess dye. Finally, the sections were dehydrated, mounted in entellan (Merck), and harden overnight.

Evaluation of the H&E staining: The degree of intestinal tissue damage was evaluated using H&E stained colonic sections. Three consecutive colonic sections were analyzed regarding specific clinically relevant parameters under the light microscope. Considered parameters were the following: thickening of the colon wall, destruction of the intestinal tissue architecture, destruction of the mucosal epithelium, infiltration of leukocytes, and depletion of goblet cells. The different parameters were assessed by optical appraisal and the first 4 parameters categorized between 0 and 3, while goblet cell depletion was categorized between 0 and 1. The overall histological score was determined by summing up the individual scores of each parameter.

Statistical analysis: All data are expressed as mean ± SEM. Statistical significance was calculated using unpaired multiple t-test (n=3) or the Mann-Whitney U test (n>3) for nonparametric distribution. Grouped data were analyzed using One- or Two-way ANOVA. All calculations were performed using GraphPad Prism 7 (GraphPad). P-values < 0.05 were considered significant.

Experimental setup for the hair growth experiments: Female C57BL/6 mice (7 weeks old) were purchased from Charles River Laboratories (Sulzfeld) and maintained under pathogen free conditions according to the institutional and national guidelines for the care and use of laboratory animals.

Removal by shaving: Dorsal neck hair of 7 w female old C57BL/6 mice was removed using a shaver. A daily dose of 100 µg sCD83 was applied i.p. from day 1 over a period of four weeks. At the end of the experiment, mice were sacrificed by cervical dislocation and dorsal skin biopsies were removed using 8 mm punches. Removal by depilation: Dorsal neck hair of 7 w female old C57BL/6 mice was removed by depilation. A daily dose of 100 µg sCD83 was applied i.p. every second day, from day 2 until day 14. At the end of the experiment, mice were sacrificed by cervical dislocation and dorsal skin biopsies were removed using 8 mm punches. Analyses of eyelashes: sCD83 (7.5 µg) was topically administered dropwise thrice a day on 7 w old female C57BL/6 mice, over a period of 14 days. After day 14, mice were sacrificed by cervical dislocation and eyelids were removed and stored in 70% EtOH for the quantification of eyelash-numbers using a microscope.

RNA extraction, cDNA synthesis and qPCR analyses (for Example 2): Biopsies were collected and stored in RNAlater (Thermo Fisher Scientific) at -80 °C. For RNA isolation the samples were thawed gently on ice and homogenization was performed in 400 µl RLT buffer (supplemented with 1% β-mercaptoethanol) using innuSPEED Lysis Tube I and SpeedMill PLUS (both from AnalytikJena). After three homogenization steps, each 20 s with 5 min cooldown at -20 °C, the suspension was centrifuged for 3 min at 16.000 g and the supernatant was collected for RNA extraction with the RNeasy Plus Mini Kit (Qiagen) according to manufacturer's protocol. cDNA synthesis was performed according to First Strand cDNA Synthesis Kit manual (Thermo Fisher Scientific). Ribosomal protein L4 was used as a housekeeping gene. The reaction and quantification was performed using a CFX96 Touch (Bio-Rad). Nucleotide sequences of the primer pairs are those provided in the respective section above.

Multi-Epitope-Ligand-Cartography (MELC): The histological MELC-technique has been applied as described before (Eckhardt, J. et al., J Histochem Cytochem 61:125-133 (2013)). Briefly, 6 mm skin biopsies were stored in Tissue-Tek^{®} O.C.T. (Sakura) at -80 °C. 5 µm slides were prepared using the Cryotome^{™} device and the fixation step was performed in acetone for 10 min at -20 °C. The sample was allowed to dry, before the residual tissue-tek^{®} was removed with 70% EtOH and the tissue sample was surrounded by a silicone ring and was washed in PBS. Staining procedure was performed using fluorescence-coupled antibodies against Foxp3 (PE), CD4 (PE), CD3e (FITC), CD45 (FITC) and Cytokeratine-14 (FITC). Propidium-iodide was used as LIVE-DEAD marker. Data were acquired using an inverted wide-field fluorescence microscope and evaluated using Image J software.

### Example 1

A. Systemic sCD83 administration promotes cutaneous wound healing: Wounds were induced in 8 weeks old C57BL/6 mice using 6 mm skin biopsy punches and wound area was assessed by caliper on day 0, 3, 6, 7 and 12. Percentage of wound closure was determined relatively to the initial value on day 0. Mice that were treated with sCD83 (i.p.) showed a significantly enhanced wound closure on day 3 and 6 when compared to mock controls (Fig. 1 A and B). These optical evaluations were supported by vimentin-specific Western blot analyses and a subsequent quantification (relatively to the β-actin control). As shown in Fig. 1 C (left-hand side), vimentin reconstitution was accelerated in sCD83-treated mice, which is associated with advanced tissue repair and wound closure. Moreover, vimentin is regarded as a regulator of fibroblast proliferation and keratinocyte differentiation (Cheng, F. et al., Proc Natl Acad Sci U S A 113:E4320-4327 (2016)), both essential cellular components of the remodeling phase.

Analyses of the cellular composition within the wound area on day 3 and 6 by flow cytometry (Fig. 1 D) revealed that neutrophil granulocytes represent the major fraction of CD45⁺ cells in sCD83- and mock-treated mice. Notoriously, neutrophils play a crucial role during wound healing by three means: (I) clearance of debris, (II) secretion of growth factors (e.g. VEGF-A) and (III) the transition towards resolution of inflammation, which is mediated by the induction of AAMs and the subsequent production of anti-inflammatory cytokines, such as IL-10 (Wang, J., Cell Tissue Res 371:531-539 (2018)). Application of sCD83 had no detectable impact on the cellular distribution on day 3. However, the decrease in neutrophilic cells numbers of sCD83-treated mice was slightly accelerated on day 6 when compared to mock controls (Fig. 1 D; day 6). In contrast, day 6 samples from sCD83-mice revealed significantly increased numbers of B lymphocytes, which were not detectable on day 3. According to the data reported by Sirbulescu *et al.,* the presence of mature B cells positively contributes to accelerated wound healing in acute and chronic wounds (Sîrbulescu, R.F. et al., Wound Repair Regen 25:774-791 (2017)). Likewise, the frequencies of T cells were significantly increased within the wound biopsies of sCD83-treated mice. Lastly, a trend towards increased monocyte and macrophage frequencies was detectable within day 6 wound biopsies of sCD83-treated mice (Fig. 1 D).

Interestingly, although no difference was detectable on cellular levels within day 3 samples of sCD83- and mock treated mice, RNA-analyses revealed a modulation of numerous wound-healing-related cytokines (Fig 2). Proinflammatory cytokines, such as IL-1β and TNFα were strikingly increased in sCD83-treated mice within the inflammatory phase on day 3. Also, VEGF was slightly upregulated on day 3. In contrast, IL-10 expression peaked on day 6 in sCD83-treated mice, thereby accelerating the transition towards resolution of inflammation and the proliferation phase. These data further support our hypothesis, that sCD83 induces IL-10-secreting AAMs. Interestingly, expression of the secretory leukocyte protease inhibitor (SLPI) was drastically upregulated in sCD83-treated mice on day 3 and moderately on day 6 (Fig. 2). As reported by Ashcroft *et al.,* SLPI is involved in the control of excessive inflammation and is therefore an important mediator for the transition towards resolution of inflammation and tissue regeneration (Ashcroft, G.S. et al., Nat Med 6:1147-1153 (2000)).

B. Topical sCD83 application also induces wound closure: Systemic drug administration may go along with several disadvantages when compared to a local - topical administration. On the one hand one cannot ensure the delivery of an intended drug concentration to the specific site of interest, thereby requiring an increased dose of medication. On the other hand, systemic drug administration can result in unexpected off-target effects, undesired cellular/ molecular interactions which subsequently could lead to adverse complications and unwanted side effects (Wen, H. et al., AAPS J 17:1327-1340 (2015)). Thus, to assess the potential topical would healing effects of sCD83, sCD83 was stabilized in a hydroxyethyl cellulose hydrogel. To ensure a uniform dosage of this sCD83-hydrogel, dorsal hair was removed by depilation. Three treatment groups were included into these *in vivo* experiments, namely: (I) topic sCD83-hydrogel or (II) saline-hydrogel applications and (III) a systemic sCD83-application, as a positive control. As expected and shown in Fig. 3 A and B, wound closure was again significantly accelerated in systemically sCD83-treated mice on day 3 and day 6. Interestingly, on day 3, wound healing was also significantly improved upon topical sCD83-admnistration, and on day 6, the topically-treated mice reached a wound closure, comparable to the systemically-treated group. These data represent the first prove, that topical sCD83 administration induces indeed an accelerated would closure.

Representative H&E stainings from day 8 skin biopsies demonstrate a restored cutaneous architecture in the systemic- but also in the topically treated sCD83 group (Fig. 3 C). Only hair regeneration, which is represented by the enlarged fat layer including numerous hair follicles, was still ongoing in the topical group. In sharp contrast, at this particular time point, granulation tissue was detectable in mock-treated mice, which is associated with an ongoing and delayed process of wound healing. Finally, in order to analyze the stability of sCD83 within the hydroxyethyl cellulose hydrogel, Western blot analyses were performed, and as shown in Fig. 3 D the sCD83 molecule was stable for at least 10 d at 4 °C. This indicates that sCD83 could be applied topically using such cellulose gels. This of course is of great interest for the development and practical application of sCD83 in future clinical trials.

C. Soluble CD83 accelerates recovery from DSS-induced colitis and fastens epithelial healing processes: Here we focused on the detailed investigation whether and how sCD83 modulates acute intestinal inflammation and induces intestinal wound healing processes as they occur in IBD pathologies. For this purpose we used the murine DSS-colitis model, whereby DSS is known to cause mucosal injuries as well as inflammation of the colon accompanied by the destruction of the colonic barrier leading to intestinal wounds (Kiesler, P. et al., Cell Mol Gastroenterol Hepatol 1:154-170 (2015); Neurath, M.F., Mucosal Immunol 7:6-19 (2014); Wirtz, S. et al., Nat Protoc 12: 1295-1309 (2017)).

Acute inflammation in the colonic intestine was established in 10-12 week old C57BL/6 mice by challenging them with 3 % DSS, supplemented in tap water, for a period of 5 days. Simultaneously, mice either received i.p. injected sCD83 or PBS for a period of 6 days. After the challenging phase, the recovery phase was initiated by replacing the DSS supplemented tap water with regular tap water. Additionally, a control group receiving neither DSS nor any other treatment regiments. Evaluating the average daily weight loss, it became evident that sCD83-treated animals showed a better regaining of weight during the recovery phase, from day 10 onwards, when compared to PBS-treated animals. Importantly, those mice receiving sCD83 reaches their initial weight by the end of the experiment, whereas the PBS group still showed a weight loss, compared to their initial weight (Fig. 4 A). To investigate possible sCD83 mediated effects on healing processes in the intestinal mucosa, different time points (d 9, d 16, d 22) were assessed. Interestingly, better recovery from colitis symptoms, within the sCD83-treated mice, was obvious on a macroscopic level, since colon shortening was reduced (Fig. 4 B). During the main recovery phase (i.e. d 16), a slightly enhanced colon length was observed in sCD83-treated mice (6.34 cm ± 0.17 cm), compared to PBS-treated mice (5.91 cm ± 0.20 cm), which was even clearer in the late recovery phase (i.e. d 22) (Fig. 1 B). The data, regarding a better wound healing during the recovery phase, were further supported by high-resolution endoscopy (Fig. 4 C). On day 16, a significantly improved recovery from colitis manifestations was observed in sCD83-treated mice, characterized by a reduced average endoscopic disease score of 7.00 ± 0.82, in comparison to PBS-treated animals having an average endoscopic disease score of 10.14 ± 0.54. In line with the above described macroscopic findings, histological evaluation of H&E stained colonic samples (d 9, d 16) provided clear evidence that the sCD83 treatment had an ameliorating effect on day 16, during the recovery phase of DSS-induced colitis (Fig. 4 D, E). At this time point sCD83-treated animals showed clearly reduced numbers of infiltrating leukocytes as well as better restitution of the epithelial barrier and the entire mucosal colonic architecture (Fig. 4 D).

Next, the RNA-expression profiles of different inflammatory cytokines, such as IFN-y, IL-1β, IL-6, and TNF-α, in the colonic tissue of sCD83-treated mice were analyzed. These investigations revealed clearly enhanced inflammatory processes within the initial recovery phase on day 9, when sCD83 was applied (Fig. 5 A). In line with these results, also factors that are known to mediate beneficial effects in intestinal wound healing processes, such as IL-17A, Cox2, CCL2, and YM-1 (gene *Chil3),* were significantly upregulated in the colonic tissue of sCD83-treated mice during the initial recovery phase (Fig. 5 A). Interestingly, during the main recovery phase on day 16, significantly reduced IL-1β expression levels were observed, in sCD83-treated animals (Fig. 5 B). A trend towards reduced expression levels were observed for all other transcripts.

D. Discussion - sCD83 boosts the inflammation phase of cutaneous wound healing: The modulatory potential of sCD83 in terms of autoimmune diseases and transplantation experiments has been reported by numerous groups. Administration of sCD83 ameliorates disease progression in murine autoimmune models e.g. EAE, SLE, IBD and AIA (Royzman, D. et al., Front Immunol 10:633 (2019); Zinser, E. et al., J Exp Med 200:345-351 (2004); Eckhardt, J. et al., Mucosal Immunol 7:1006-1018 (2014); Starke, C. et al., Immunobiology 218:1411-1415 (2013)). Analogously, sCD83 has significantly improved graft survival in cornea-, cardiac-, renal-, and skin transplantation models (Ge, W. et al., Transplantation 90:1145-1156 (2010); Xu, J.F. et al., Transpl Int 20:266-276 (2007); Lan, Z. et al., Transplantation 90:1278-1285 (2010)). Mechanistically by the IDO-mediated induction of Treg differentiation and proliferation [30]. Within the present study we report for the very first time the striking effects of sCD83-treatment on cutaneous and intestinal wound healing.

Cutaneous wound healing was significantly enhanced in systemically sCD83-treated mice, which was accompanied by an increased deposition of vimentin within the 8 mm punches on day 3 and day 6. On the one hand vimentin is an intermediate filament, mainly expressed in mesenchymal stem cells and thus enhances the reconstitution of wound area (Walker, J.L. et al., Proc Natl Acad Sci U S A 107:13730-13735 (2010)). On the other hand, vimentin provides crucial stimuli for fibroblast proliferation and the differentiation of keratinocytes; thereby further enhancing the healing capacities of cutaneous tissue in the course of reepithelization and remodeling (Cheng, F. et al., Proc Natl Acad Sci U S A 113:E4320-4327 (2016)).

On day 6, but not on day 3, a different cell composition was detected between sCD83- and mock-treated mice. A prominent accumulation of B220⁺ B cells was observed in sCD83-treated mice, and although, little is known regarding the role of B cells during the course of wound healing, it has been reported, that wound closure is accelerated in the presence of mature B cells (Sirbulescu, R.F. et al., Wound Repair Regen 25:774-791 (2017)). In addition, the presence of CD3⁺ T cells on day 6 is associated with enhanced tissue regeneration, e.g. via the secretion of IL-22, particularly by Th17 (Brockmann, L. et al., Int J Mol Sci 18 (2017)). Noteworthy, IL-17A-expression was enhanced within the colonic tissue of sCD83-treated DSS-mice on day 9, indicating ameliorating effects of sCD83 via Th17-mediated immune responses. In this respect it is important to know that, in sharp contrast to inflammatory autoimmune disorders, where the pathogenesis is based on excessive T cell activity and cytokine secretion (McInnes, I.B. and Schett, G., N Engl J Med 365:2205-2219 (2011)), a temporary upregulation of proinflammatory mediators is crucial for proper wound closure (Hubner, G. et al., Cytokine 8:548-556 (1996)). Generally spoken, wound healing is initiated by a strong local inflammation, which, as represented by increased levels of proinflammatory cytokines, is strikingly enhanced in sCD83-treated mice. Herein, IL-1β and TNF α play a pivot role in the course of wound healing and their blockade, either by knockout or depletion results in an impaired inflammation phase and poor clearance of infectious agents (Ritsu, M. et al., J Dermatology & Dermatologic Surgery 21:14-19 (2017); Ishida, Y. et al., J Immunol 176:5598-5606 (2006)). Additionally, the observed increased secretion of SLPI in sCD83-treated mice, has been described to be associated with anti-microbial properties and the amelioration of excessive inflammation (Jin, F.Y. et al., Cell 88:417-426 (1997)). SLPI knockout experiments revealed a drastically impaired wound closure due to impaired resolution of inflammation and wound chronification (Ashcroft, G.S. et al., Nat Med 6:1147-1153 (2000)). However, the major role of SLPI lies in the inhibition of elastase activity upon transition to the proliferation / remodeling phases, since excessive protease activity is associated with age-related complications of wound healing (Herrick, S. et al., Lab Invest 77:281-288 (1997)).

Therefore, we hypothesize that the administration of sCD83 overwrites this age-related setting and restores the healing capacities, e.g. in elderly or diabetic patients, at least temporary.

Interestingly, although sCD83-treatment was continued until the end of the experiment, the expression of proinflammatory cytokines was diminished around day 6, thereby indicating a manifold regulated and long-term modulation by sCD83 throughout the healing process, and does not simply rely on boosting IL-1β and TNF α expression. This critical modulation by sCD83 is further supported by the delayed induction of IL-10 on day 6, which represents the induction of alternatively activated macrophages and the subsequent transition towards proliferation and reepithelization. The shift towards AAMs was most likely mediated by the clearance process of apoptotic neutrophils, which initiates a so called "feed-forward pro-resolution program" in macrophages and the subsequent production of IL-10 (Wang, J., Cell Tissue Res 371:531-539 (2018)). Finally, the slightly increased VEGF levels may positively contribute to angiogenesis and sufficient supply of the wound area with oxygen and nutritions, since malnutrition of the wound area is associated with delayed wound closure for instance in elderly patients (Sgonc, R. and Gruber, J., Gerontology 59:159-164 (2013)).

All these data indicate that the role of sCD83 does not just rely on a unique activation/ modulation on a single cell level but modifies wound healing by multifactorial steps. Initially it boosts the inflammatory phase and thereby bacterial clearance without disturbing the natural kinetics of wound healing. Herein we see a profound advantage of our sCD83-therapy in comparison to commonly used medications (some of them immunosuppressive), since wound healing is a tightly regulated process, where mechanistic deviations can result in delayed wound closure or the development of chronic wounds. This is one reason, why numerous therapeutic agents reportedly provide a negative impact on wound healing (Guo, S. and Dipietro, L.A.., J Dent Res 89:219-229 (2010); Krischak, G.D. et al., J Wound Care 16:76-78 (2007)). In this context, especially the medication of autoimmune diseases is highly controversial, as the administration of e.g. methotrexate or corticosteroids is often associated with delayed wound healing, scar formation, postoperative complications and ulcerations (Tekur, V.K., Indian Dermatol Online J 7:418-420 (2016)). This is most likely due to the interference with at least one of the manifold mechanisms of wound healing, e.g. inflammatory response or cell proliferation (Anstead, G.M., Adv Wound Care 11:277-285 (1998)). This is obviously not the case when sCD83 is used, since our previous transplantation studies revealed no negative effects on wound healing. On the contrary, transplant survival was prolonged and wound healing accelerated (Ge, W. et al., Transplantation 90:1145-1156 (2010); Bock, F. et al., J Immunol 191:1965-1975 (2013)). Thus, in contrast to currently approved immunosuppressant drugs, such as rapamycin, no adverse events were observed during sCD83-therapy in transplantation experiments, regarding wound healing. Since delayed wound healing is a major cause of transplant morbidity (Ueno, P. et al., Transplantation 101:844-850 (2017); Mehrabi, A. et al., Clin Transplant 20(Suppl 17):97-110 (2006)), sCD83 therapy opens new strategies to overcome these intrinsic problems of currently available and administered drugs.

Of major practical interest is also the finding, that topical administration of sCD83 potently improved cutaneous wound closure. Although no severe side effects of sCD83 therapy were reported to date, a locally-restricted medication provides crucial advantages over the systemic administration, namely an optimal drug supply and a restricted risk of systemic side-effects. Within our cutaneous wound healing experiments, topical sCD83 uptake is ensured by the mechanical disruption of epithelial barriers and can therefore be proposed as a first aid wound care medication, which sterilizes the wound area by enhanced proinflammatory cytokine production, and subsequently increased frequencies of immune cells which accelerate would healing and tissue remodeling. Noteworthy is also the fact, that sCD83 remained stable and active for at least 10 days at 4 °C, in the used hydrogel matrix and can therefore most likely be stored for long-term treatment. This will be evaluated in further studies.

A balanced release of pro- and anti-inflammatory cytokines and mediators is the main source of regulating intestinal immune reactions against potentially noxious microbial components as well as innocuous commensal bacteria. Intensive research has shown that in IBD pathologies intestinal immune reactions become decontrolled upon activation by luminal antigens, which are able to cross an attenuated epithelial barrier. In return, this causes severe mucosal inflammation which goes along with continuous structural destruction of the epithelial tissue (Zundler, S. et al., Gut 68:1688-1700 (2019); Strober, W. and Fuss, I.J., Gastroenterology 140:1756-1767 (2011). In addition to the fundamental goal of preventing onset of colitis, recent therapy goals of IBD have also focused on improving the healing process of the inflamed mucosa upon disruption.

In addition to cutaneous wound healing, findings within the DSS-induced colitis experiments of this study have shown that sCD83 also has a beneficial effect on the repair mechanisms of the intestinal epithelium, thereby favoring an accelerated recovery from colitis symptoms. When analyzing clinically relevant parameters for the IBD pathologies, results revealed that in sCD83-treated animals the main recovery phase (d 16) was characterized by clearly accelerated regaining of weight combined with a marginally improved colon length, which became explicitly obvious towards the late recovery phase. Corresponding endoscopic and histological analyses confirmed the positive properties of sCD83 specifically during the main recovery phase in the DSS-induced colitis model.

Further gene expression analyses of colon tissue focused either on pro-inflammatory mediators such as IFN-γ, IL1-β, IL-6, and TNF-α or factors which are classically associated with inflammation but also provide beneficial effects in healing processes such as IL-17A, Cox2, CCL2, and YM-1. In colon samples from sCD83-treated mice expression levels of the pro-inflammatory mediators were highly elevated during the initial recovery phase and almost rebounded to their underlying by the time the mice reached the main recovery phase. Contrary to that, PBS-treated mice exhibited only slightly elevated levels of those factors in the initial recovery phase, which either became stagnant or continued to increase during the main recovery phase. These results suggest that sCD83 affects certain components of the mucosal environment to induce higher levels of pro-inflammatory cytokines in the initial recovery phase. This establishment of an exceptionally intense pro-inflammatory status might in turn result in faster induction and more effective wound healing processes in the later recovery phase which also correlates with the results from macroscopic and endoscopic evaluation of the colon. Supporting our conclusion, other groups already showed an essential role of specific cytokines such as IFN-γ and IL-1β right upon a mucosal injury as those factors regulate the crucial step of epithelial restitution by positively promote TGF-β production in IECs (Neurath, M.F., Mucosal Immunol 7:6-19 (2014); Dignass, A.U. and Podolsky, D.K., Gastroenterology 105:1323-1332 (1993)). Results from former studies also suggest a correlation between the release of IFN-y and IDO expression in DCs, followed by the induction of tolerogenic mechanisms (Pallotta, M.T. et al., Nat Immunol 12:870-878 (2011); Chen, W., Nat Immunol 12:809-811 (2011)). In agreement with these reports we observed highly elevated levels of IDO expression in sCD83-treated animals during the initial recovery phase of the DSS-induced colitis model (data not shown). Supporting our results IDO-mediated kynurenine is proposed to contribute to the induction of long term tolerance and mucosal wound repair mechanisms (Chen, W., Nat Immunol 12:809-811 (2011); Sugihara, K. et al., Front Immunol 9:3183 (2018)).

Comparable to the analyzed pro-inflammatory mediators, interestingly also the factors IL-17A, Cox2, CCL2 and YM-1 showed this striking increased expression levels during the initial recovery phase. When focusing on the main recovery phase, in which intestinal wound healing processes mainly occur, a similar picture as for the pro-inflammatory cytokines was given. In sCD83-treated mice the expression of most factors drop back towards their basis level whereas in PBS-treated animals almost no modulation was detectable. As already mentioned earlier, most of the analyzed factors are not known for its beneficial effects in regard to wound healing processes in a classical way and, indeed, are controversially discussed in this regard. However, it is further known that a solid initial inflammatory phase is necessary to eliminate the pathogenic agents for efficient wound healing. Thus, a potential mechanistic effect of sCD83 may be the induction of an increased expression of inflammatory mediators at the initial recovery phase, which not only positively modulates the mucosal epithelium itself, but also the bacterial killing efficiency of phagocytic cell populations. In return, this provides a basis for a faster transition into following wound healing processes. Supporting our conclusion, analyses of colon and ileum samples of IBD patients showed that the inducible enzyme Cox2 is, besides its expression in cell types such as macrophages and fibroblasts during intestinal inflammation, also expressed by IECs, whereas it is not detectable in healthy patients (Neurath, M., Nat Rev Gastroenterol Hepatol 14:688 (2017)). Moreover, it has been reported that the selective inhibition of Cox2 in TNBS-induced colitis in rats leads to exacerbated colitis symptoms and epithelial destruction, which has been associated with the absence of prostaglandins that are normally produced by Cox2 (Singer, II et al., Gastroenterology 115:297-306 (1998); Reuter, B.K. et al., J Clin Invest 98:2076-2085 (1996)). Besides Cox2, a recent study showed that the chemokine CCL2 supports the activation of inflammatory macrophages and thereby mediates inflammatory responses (Carson, W.F.t. et al., Cell Immunol 314:63-72 (2017)). Simultaneously to the induction of a solid inflammatory immune response, sCD83 can support a quicker intestinal wound closure by mediating increased epithelial cell proliferation. In this regard, studies have also shown that the cytokines IL-6 and IL-17A when tightly controlled are suggested to mediate tissue repair and protective mechanisms, as they boost epithelial cell proliferation and survival at certain recovery phases (Neurath, M.F., Mucosal Immunol 7:6-19 (2014); Brockmann, L. et al., Int J Mol Sci 18 (2017)).

In summary, our results provide strong evidence that sCD83 is an interesting biological agent for the treatment of e.g. hard-to-heal wounds in the context of cutaneous and mucosal injuries and/ or individuals receiving immunosuppressive therapy, which is accompanied with delayed wound closure and an increased susceptibility to infections.

### Example 2

The soluble CD83 molecule induces hair growth. In addition to the impact of sCD83 on wound healing described in Example 1 above, an accelerated hair growth in sCD83-treated mice in four independent experimental settings was observed.
A. sCD83 accelerated hair regrowth after excessive barbering: in sCD83-treated C57BL/6 mice hair regrowth was significantly boosted within the dorsal region at day 21 (Fig. 6A). The area of bald sites was quantified relatively to the whole body area and revealed a significant recovery from hair loss (Fig. 6B).

In order to get further insights regarding the underlying mechanisms of sCD83 induced hair regrowth, we have analyzed the presence of regulatory T cells (Tregs) in dorsal skin biopsies, 31 days after depilation. As reported by the group of Rosenblum, the presence of Tregs within the sites of hair follicles in the skin, is associated with enhanced hair growth. In sharp contrast, the ablation of Tregs resulted in impaired anagen induction and hair regrowth (Ali, N. et al., Cell 169:1119-1129 e1111 (2017)) Since it is known, that sCD83 induces Tregs (Bock, F. et al., J Immunol 191:1965-1975 (2013)), we hypothesized that sCD83 increases the numbers of Tregs in treated animals and that this would lead to a better hair growth. Indeed, MELC-analyses of skin samples from sCD83 treated animals revealed an accumulation of CD4⁺/Foxp3⁺ Tregs in sCD83-treated mice when compared to the mock-group (Fig. 7 A; 20 Tregs vs. 11 Tregs). These data were further supported by qPCR analyses of the skin, which revealed a significantly increased expression of *Foxp3* in the sCD83 group (Fig. 7 B).
B. sCD83 promotes hair regrowth after wound induction: in order to investigate the effects of sCD83 on hair regrowth after wound infliction, dorsal neck region was depilated right before the induction of 6 mm wounds. In the context of hair growth, depilation is a potent stimulus for telogen to anagen transition and the subsequent induction of hair growth (Muller-Rover, S. et al., J Invest Dermatol 117:3-15 (2001)). Interestingly, mice that received sCD83 i.p., revealed an enhanced coloration of the dorsal skin, which according to Muller-Rover *et al.* is associated with a progressed phase of hair growth within the anaphase (Muller-Rover, S. et al., J Invest Dermatol 117:3-15 (2001)) (Fig. 8). Thereby, the dark shape of dorsal skin is associated with increased melanin production within the hair follicles (Plikus, M.V. and Chuong, C.M., J Invest Dermatol 128:1071-1080 (2008)), which was obviously not the case in mock-treated mice.
C. sCD83 promotes hair regrowth after depilation: in order to substantiate the effect of sCD83 on hair growth, the dorsal neck hair of healthy C57BL/6 mice was either removed by depilation or shaving, without any additional experimental procedures. As mentioned afore, depilation induces a telogen to anagen transition. Thus, depilated mice were systemically treated with sCD83 and hair growth was assessed by macroscopic evaluation until day 14. As depicted in Fig. 9 hair regrowth was induced in depilated sCD83- as well as in mock-treated mice, however, hair regrowth was denser in sCD83-mice when compared to mock-treated mice (indicated by red circles).
D. sCD83 increases the number and length of eyelashes after topical sCD83 application: Finally, we investigated if local administration of sCD83 modulates the number of eyelashes. Therefore, sCD83 was topically administered in the form of eye drops (7 µg sCD83) thrice a day for a time period of 14 days. Eyelids were removed on day 14 and the number of eyelashes was determined under magnification by two independent investigators. Surprisingly and noteworthy, the sCD83-treated group revealed increased numbers of eyelashes with a longer and thicker shape, when compared with mock-controls (Fig. 10).
E. Discussion: Taken together, the above described independent experiments clearly support the effect of sCD83 on hair growth. Our results further demonstrate that the accumulation of Tregs in the skin tissue is significantly increased by sCD83-treatment. As reported by Ali et al., Tregs positively contribute to the induction of the anagen phase and subsequent hair regrowth (Ali, N. et al., Cell 169:1119-1129 e1111 (2017)). Noteworthy, topical administration of sCD83 onto the eye boosted the formation of eyelashes. In summary, the above described experimental data show for the first that sCD83 improves hair regrowth and that topical administration increases the numbers of eyelashes.

Thus, sCD83 represents a highly interesting biological agent to promote hair regrowth, e.g. in individuals suffering from age or disease related hair loss or patients treated with chemotherapy, which also results in pronounced baldness (effluvium).

### Example 3

Expression of sCD83 in *Pichia pastoris* flask cultures: A flowchart of sCD83 production is depicted in Fig. 11. Briefly, N6HIS-sCD83-pPIC9K transformed P. *pastoris* (strain: GS115) cells were cultured for 72 h/30 °C and 150 rpm in a shaking incubator (the sequence of N6HIS-sCD83 being shown below). After 72 h, OD₆₀₀ was determined by photometer and centrifuged for 5 min at 3.000xg/4 °C. The supernatant was completely decanted and the pellet was divided into four shaking flasks, each with 400 ml BMMY and a final optical density of OD₆₀₀ = 10. Yeast culture was incubated for additional 72 h/30 °C and 150 rpm with a daily supplementation of 1% methanol. Finally, the cell suspension was harvested by centrifugation (5.000xg; 10 min; 4 °C). The supernatant was filtered through 0.22 µm stericups for the subsequent purification of his-tagged proteins by highpressure liquid chromatography (HPLC) using HisPur^{™} Ni-NTA chromatography cartridges. A maximal volume of 150 ml supernatant was loaded at once onto a superloop column and transferred to the 5 ml HisPur^{™} Ni-NTA column at a flow rate of 5 ml/min (pressure must not exceed a maximal value of 0.3 MPa). His-tagged sCD83 was retained within the HisPur^{™} Ni-NTA column, while other molecules passed the resin and proceeded with the flow-through. Afterwards, the HisPur^{™} Ni-NTA column was rinsed with 60 ml Equilibration Buffer. For sCD83 elution, elution buffer was used and the protein solution was collected in a clean syringe as soon as the OD has reached a value of 1.000 on the y-axis. The sCD83 solution was stored overnight at 4 °C. The next day, imidazole was removed by HiPrep desalting procedure. For this purpose, the protein solution was again loaded onto the superloop and transferred to the HiPrep column with a flow rate of 3 ml/min. Afterwards the protein was eluted in commercially available PBS. Lastly, possible LPS contaminations were removed by centrifugation in Vivapure Q Maxi H columns for 5 min at 1500 rpm/4 °C, before sCD83 solution was filtered (0.22 µm). Aliquots were stored at - 80 °C. Concentration of sCD83 was determination by BCA method according to manufacturer's instructions.

Sequence of N6HIS-sCD83 (SEQ ID NO:11):

6xHIS-tag
TACCATCATCACCACCACCATACGCCGGAGGTGAAGGTGGCTTGCTCCGAAGATGTGG
ACTTGCCCTGCACCGCCCCCTGGGATCCGCAGGTTCCCTACACGGTCTCCTGGGTCAA
GTTATTGGAGGGTGGTGAAGAGAGGATGGAGACACCCCAGGAAGACCACCTCAGGGG
ACAGCACTATCATCAGAAGGGGCAAAATGGTTCTTTCGACGCCCCCAATGAAAGGCCCT
ATTCCCTGAAGATCCGAAACACTACCAGCTGCAACTCGGGGACATACAGGTGCACTCTG
CAGGACCCGGATGGGCAGAGAAACCTAAGTGGCAAGGTGATCTTGAGAGTGACAGGAT
GCCCTGCACAGCGTAAAGAAGAGACTTTTAAGAAATACAGAGCGGAGATTTGA
Stop codon

The Start codon is provided by the pPIC9K-expression vector. More details about the expression vector and the processing of the sCD83 are provided in Fig. 12.

### Example 4

A. Treatment with sCD83 protein improved cutaneous wound healing significantly better than with the Fc-CD83 fusion protein: Wounds in seven-week-old female C57BL/6 mice were inflicted using a 6 mm biopsy punch. Mice were systemically treated using a daily dose of 100 µg commercially available recombinant Fc-tagged human sCD83 fusion protein, from Creative Biomart - CD83-150H (description of CD83-150H fusion protein: The extracellular domain of human CD83 (aa 20-131 of SEQ ID NO:2) is fused to the N-terminus of the Fc region of human IgG1 with the sequence of SEQ ID NO: 50), sCD83 (description of sequence: sCD83 sequence: aa 20-145 of SEQ ID NO:2), or saline. Wound area was measured on day 0, 3 and 6 using a caliper and wound closure was determined for day 3 and 6. Percentage of wound closure was determined relatively to the initial value on day 0. Mice which have been treated with sCD83 (i.p.) showed a significantly enhanced wound closure on day 3 and 6 when compared to the CD83-Fc fusion protein, and mock controls. The results are shown in Fig. 13, data are shown as mean with single values ± SEM. Statistical significance was evaluated using two-way ANOVA. Asterisks mark statistically significant difference (*p < 0.05, **p < 0.01, ***p < 0.001, and ****p < 0.0001) while "ns" indicates that there is no statistical significance. This shows that the sCD83 molecule induces a statistically significant better cutaneous wound healing process when compared to CD83-Fc fusion protein.
B. Discussion: In summary, the above described experiments clearly show, that sCD83 leads to a statistically significant better wound healing processes, compared to a CD83-Fc fusion protein.

## Claims

1. A soluble form of a member of the CD83 family of proteins consisting of the extracellular domain of the CD83 protein with the amino acid residues 20 to 144 of SEQ ID NO:2 (sCD83 protein), a functional derivative of said sCD83 protein having up to 10 amino acids attached to the C- or N-terminus of said sCD83 protein and/or having up to 10 amino acids of said sCD83 protein substituted, a dimeric or trimeric form of said sCD83 protein, or a dimeric or trimeric form of the functional derivative of said sCD83 protein, for use in wound closure of cutaneous wounds.

2. The sCD83 protein, the functional derivative of said sCD83 protein, the dimeric or trimeric form of said sCD83 protein, or the dimeric or trimeric form of the functional derivative of said sCD83 protein for use in wound closure of cutaneous wounds of claim 1, wherein said sCD83 protein
(i) consists of amino acid residues 20 to 144 of SEQ ID NO:2; or
(ii) has up to 10 amino acid residues derived from the neighboring intracellular domain attached to its C-terminus, preferably the soluble CD83 protein consists of amino acid residues 20 to 145 of SEQ ID NO:2; or
(iii) has functional sequences of up to 10 amino acid residues attached to its N-terminus, and preferably said sCD83 protein carries at the N-terminus the additional amino acids Gly-Ser-Pro-Gly or Tyr-His-His-His-His-His-His.

3. The sCD83 protein, the functional derivative of said sCD83 protein, the dimeric or trimeric form of said sCD83 protein, or the dimeric or trimeric form of the functional derivative of said sCD83 protein for use in wound closure of cutaneous wounds of claim 1, wherein the sCD83 protein is a dimer or trimer, preferably a homodimer connected through one or more of the cysteine residues of the monomeric form of the sCD83 protein, most preferably the sCD83 protein is as defined in claim 2 and/or the homodimer is connected through the fifth cysteine residue of the monomeric form of the sCD83 protein.

4. The sCD83 protein, the functional derivative of said sCD83 protein, the dimeric or trimeric form of said sCD83 protein, or the dimeric or trimeric form of the functional derivative of said sCD83 protein for use in wound closure of cutaneous wounds of claim 1, wherein the sCD83 protein is a monomeric CD83 protein, preferably a monomeric CD83 protein where one or more of the cysteine residues have been substituted by same or different small and/or polar amino acid residues selected from serine, alanine and glycine, most preferably
(i) the sCD83 protein is as defined in claims 2 or 3; or
(ii) one cysteine residue, notably the fifth cysteine residue has been replaced; or
(iii) the sCD83 protein consists of amino acid residues 20 to 144 of SEQ ID NO:2, where the cysteine residue at position 129 has been replaced by a serine residue; or
(iv) the sCD83 protein consists of amino acid residues 20 to 144 of SEQ ID NO:2, where the cysteine residue at position 129 has been replaced by a serine residue, and further carries at the N-terminus the additional amino acids Gly-Ser-Pro-Gly.

5. The sCD83 protein, the functional derivative of said sCD83 protein, the dimeric or trimeric form of said sCD83 protein, or the dimeric or trimeric form of the functional derivative of said sCD83 protein for use in wound closure of cutaneous wounds of claim 1, wherein the sCD83 protein has a native glycosylation pattern or is non-glycosylated.

6. The sCD83 protein, the functional derivative of said sCD83 protein, the dimeric or trimeric form of said sCD83 protein, or the dimeric or trimeric form of the functional derivative of said sCD83 protein for use in wound closure of cutaneous wounds of any one of claims 1 to 5, wherein the wound healing comprises
(a) systemic administration of the sCD83 protein, the functional derivative of said sCD83 protein, the dimeric or trimeric form of said sCD83 protein, or the dimeric or trimeric form of the functional derivative of said sCD83 protein for accelerated wound closure of cutaneous wounds; or
(b) topical administration of the sCD83 protein, the functional derivative of said sCD83 protein, the dimeric or trimeric form of said sCD83 protein, or the dimeric or trimeric form of the functional derivative of said sCD83 protein, preferably in form of a gel-based sCD83 solution, for wound closure of skin lesions, including locally restricted wounds and chronic wounds.

7. A soluble form of a member of the CD83 family of proteins consisting of the extracellular domain of the CD83 protein with the amino acid residues 20 to 144 of SEQ ID NO:2 (sCD83 protein), a functional derivative of having up to 10 amino acids attached to the C- or N-terminus of said sCD83 protein and/or having up to 10 amino acids of said sCD83 substituted, a dimeric or trimeric form of said sCD83 protein, or a dimeric or trimeric form of the functional derivative of said sCD83 protein, for use in therapeutic hair growth after physical stress/medication and/or disease.

8. The sCD83 protein, the functional derivative of said sCD83 protein, the dimeric or trimeric form of said sCD83 protein, or the dimeric or trimeric form of the functional derivative of said sCD83 protein for use in therapeutic hair growth of claim 7, wherein said sCD83 protein
(i) consists of amino acid residues 20 to 144 of SEQ ID NO:2; or
(ii) has up to ten amino acid residues derived from the neighboring intracellular domain attached to its C-terminus, preferably the soluble CD83 protein consists of amino acid residues 20 to 145 of SEQ ID NO:2; or
(iii) has functional sequences of up to 10 amino acid residues attached to its N-terminus, and preferably said sCD83 protein carries at the N-terminus the additional amino acids Gly-Ser-Pro-Gly or Tyr-His-His-His-His-His-His.

9. The sCD83 protein, the functional derivative of said sCD83 protein, the dimeric or trimeric form of said sCD83 protein, or the dimeric or trimeric form of the functional derivative of said sCD83 protein for use in therapeutic hair growth of claim 7, wherein the sCD83 protein is a dimer or trimer, preferably a homodimer connected through one or more of the cysteine residues of the monomeric form of the sCD83 protein, most preferably the sCD83 protein is as defined in claim 8 and/or the homodimer is connected through the fifth cysteine residue of the monomeric form of the sCD83 protein.

10. The sCD83 protein, the functional derivative of said sCD83 protein, the dimeric or trimeric form of said sCD83 protein, or the dimeric or trimeric form of the functional derivative of said sCD83 protein for use in therapeutic hair growthof claim 7, wherein the sCD83 protein is a monomeric CD83 protein, preferably a monomeric CD83 protein where one or more of the cysteine residues have been substituted by same or different small and/or polar amino acid residues selected from serine, alanine, and glycine, most preferably
(i) the sCD83 protein is as defined in claims 8 or 9; or
(ii) one cysteine residue, notably the fifth cysteine residue has been replaced; or
(iii) the sCD83 protein consists of amino acid residues 20 to 144 of SEQ ID NO:2, where the cysteine residue at position 129 has been replaced by a serine residue; or
(iv) the sCD83 protein consists of amino acid residues 20 to 144 of SEQ ID NO:2, where the cysteine residue at position 129 has been replaced by a serine residue, and further carries at the N-terminus the additional amino acids Gly-Ser-Pro-Gly.

11. The sCD83 protein, the functional derivative of said sCD83 protein, the dimeric or trimeric form of said sCD83 protein, or the dimeric or trimeric form of the functional derivative of said sCD83 protein for use in therapeutic hair growth of claim 7, wherein the sCD83 protein has a native glycosylation pattern or is non-glycosylated.

12. The sCD83 protein, the functional derivative of said sCD83 protein, the dimeric or trimeric form of said sCD83 protein, or the dimeric or trimeric form of the functional derivative of said sCD83 protein for use in therapeutic hair growth of any one of claims 7 to 11, wherein the therapeutic hair growth comprises systemic or topical sCD83 application after physical stress/medication and/or disease.

13. A composition comprising a soluble form of a member of the CD83 family of proteins consisting of the extracellular domain of the CD83 protein with the amino acid residues 20 to 144 of SEQ ID NO:2 (sCD83 protein), a functional derivative of said sCD83 protein having up to 10 amino acids attached to the C- or N-terminus of said sCD83 protein and/or having up to 10 amino acids of said sCD83 protein substituted, a dimeric or trimeric form of said sCD83 protein, or a dimeric or trimeric form of the functional derivative of said sCD83 protein, for use in therapeutic hair growth after physical stress/medication and/or disease.

14. The composition for use in therapeutic hair growth of claim 13, wherein
(i) the sCD83 protein is as defined in claims 8 to 11; and/or
(ii) the therapeutic hair growth comprises systemic or topical sCD83 application after physical stress/medication and/or disease.

15. A cosmetic non-therapeutic method for the acceleration of hair growth and for hair care, which method comprises systemically or topically applying a composition comprising a soluble form of a member of the CD83 family of proteins consisting of the extracellular domain of the CD83 protein with the amino acid residues 20 to 144 of SEQ ID NO:2 (sCD83 protein), a functional derivative of said sCD83 protein having up to 10 amino acids attached to the C- or N-terminus of said sCD83 protein and/or having up to 10 amino acids of said sCD83 protein substituted, a dimeric or trimeric form of said sCD83 protein, or a dimeric or trimeric form of the functional derivative of said sCD83 protein to the human or animal skin or hair.

16. The cosmetic non-therapeutic method of claim 15, wherein the sCD83 protein is as defined in claims 8 to 11.

## Patentansprüche

1. Lösliche Form eines Vertreters der CD83-Proteinfamilie, der aus der extrazellulären Domäne des CD83-Proteins mit den Aminosäureresten 20 bis 144 von SEQ ID Nr. 2 besteht (sCD83-Protein), eines funktionellen Derivats des sCD83-Proteins mit bis zu 10 Aminosäuren, die an den C- oder N-Terminus des sCD83-Proteins gebunden sind, und/oder bei dem bis zu 10 Aminosäuren des sCD83-Proteins substituiert sind, einer dimeren oder trimeren Form des sCD83-Proteins oder einer dimeren oder trimeren Form des funktionellen Derivats des sCD83-Proteins zur Verwendung beim Wundverschluss von Hautwunden.

2. sCD83-Protein, funktionelles Derivat des sCD83-Proteins, dimere oder trimere Form des sCD83-Proteins oder dimere oder trimere Form des funktionellen Derivats des sCD83-Proteins zur Verwendung beim Wundverschluss von Hautwunden gemäß Anspruch 1, wobei das sCD83-Protein
(i) aus den Aminosäureresten 20 bis 144 von SEQ ID Nr. 2 besteht; oder
(ii) bis zu 10 Aminosäurereste aufweist, die von der benachbarten intrazellulären Domäne, die an seinen C-Terminus gebunden ist, abgeleitet sind, wobei vorzugsweise das lösliche CD83-Protein aus den Aminosäureresten 20 bis 145 von SEQ ID Nr. 2 besteht; oder
(iii) funktionelle Sequenzen von bis zu 10 Aminosäureresten aufweist, die an seinen N-Terminus gebunden sind, und vorzugsweise das sCD83-Protein am N-Terminus die zusätzlichen Aminosäuren Gly-Ser-Pro-Gly oder Tyr-His-His-His-His-His-His trägt.

3. sCD83-Protein, funktionelles Derivat des sCD83-Proteins, dimere oder trimere Form des sCD83-Proteins oder dimere oder trimere Form des funktionellen Derivats des sCD83-Proteins zur Verwendung beim Wundverschluss von Hautwunden gemäß Anspruch 1, wobei das sCD83-Protein ein Dimer oder Trimer ist, vorzugsweise ein Homodimer, das über einen oder mehrere der Cysteinreste der monomeren Form des sCD83-Proteins verbunden ist, am meisten bevorzugt das sCD83-Protein wie in Anspruch 2 definiert ist und/oder das Homodimer über den fünften Cysteinrest der monomeren Form des sCD83-Proteins verbunden ist.

4. sCD83-Protein, funktionelles Derivat des sCD83-Proteins, dimere oder trimere Form des sCD83-Proteins oder dimere oder trimere Form des funktionellen Derivats des sCD83-Proteins zur Verwendung beim Wundverschluss von Hautwunden gemäß Anspruch 1, wobei das sCD83-Protein ein monomeres CD83-Protein ist, vorzugsweise ein monomeres CD83-Protein, bei dem einer oder mehrere der Cysteinreste durch gleiche oder verschiedene kleine und/oder polare Aminosäurereste, die aus Serin, Alanin und Glycin ausgewählt sind, substituiert sind, wobei am meisten bevorzugt
(i) das sCD83-Protein wie in den Ansprüchen 2 oder 3 definiert ist; oder
(ii) genau ein Cysteinrest, insbesondere der fünfte Cysteinrest, ersetzt ist; oder
(iii) das sCD83-Protein aus den Aminosäureresten 20 bis 144 von SEQ ID Nr. 2 besteht, wobei der Cysteinrest auf Position 129 durch einen Serinrest ersetzt ist; oder
(iv) das sCD83-Protein aus den Aminosäureresten 20 bis 144 von SEQ ID Nr. 2 besteht, wobei der Cysteinrest auf Position 129 durch einen Serinrest ersetzt ist und weiterhin am N-Terminus die zusätzlichen Aminosäuren Gly-Ser-Pro-Gly trägt.

5. sCD83-Protein, funktionelles Derivat des sCD83-Proteins, dimere oder trimere Form des sCD83-Proteins oder dimere oder trimere Form des funktionellen Derivats des sCD83-Proteins zur Verwendung beim Wundverschluss von Hautwunden gemäß Anspruch 1, wobei das sCD83-Protein ein natives Glycosylierungsmuster aufweist oder nicht glycosyliert ist.

6. sCD83-Protein, funktionelles Derivat des sCD83-Proteins, dimere oder trimere Form des sCD83-Proteins oder dimere oder trimere Form des funktionellen Derivats des sCD83-Proteins zur Verwendung beim Wundverschluss von Hautwunden gemäß einem der Ansprüche 1 bis 5, wobei die Wundheilung Folgendes umfasst:
(a) die systemische Verabreichung des sCD83-Proteins, des funktionellen Derivats des sCD83-Proteins, der dimeren oder trimeren Form des sCD83-Proteins oder der dimeren oder trimeren Form des funktionellen Derivats des sCD83-Proteins zum beschleunigten Wundverschluss von Hautwunden; oder
(b) die topische Verabreichung des sCD83-Proteins, des funktionellen Derivats des sCD83-Proteins, der dimeren oder trimeren Form des sCD83-Proteins oder der dimeren oder trimeren Form des funktionellen Derivats des sCD83-Proteins, vorzugsweise in Form einer sCD83-Lösung auf Gelbasis, zum Wundverschluss von Hautläsionen einschließlich lokal begrenzter Wunden und chronischer Wunden.

7. Lösliche Form eines Vertreters der CD83-Proteinfamilie, der aus der extrazellulären Domäne des CD83-Proteins mit den Aminosäureresten 20 bis 144 von SEQ ID Nr. 2 besteht (sCD83-Protein), funktionelles Derivat mit bis zu 10 Aminosäuren, die an den C- oder N-Terminus des sCD83-Proteins gebunden sind, und/oder bei dem bis zu 10 Aminosäuren des sCD83-Proteins substituiert sind, dimere oder trimere Form des sCD83-Proteins oder dimere oder trimere Form des funktionellen Derivats des sCD83-Proteins zur Verwendung bei therapeutischem Haarwachstum nach physischem Stress/Medikation und/oder Krankheit.

8. sCD83-Protein, funktionelles Derivat des sCD83-Proteins, dimere oder trimere Form des sCD83-Proteins oder dimere oder trimere Form des funktionellen Derivats des sCD83-Proteins zur Verwendung bei therapeutischem Haarwachstum gemäß Anspruch 7, wobei das sCD83-Protein
(i) aus den Aminosäureresten 20 bis 144 von SEQ ID Nr. 2 besteht; oder
(ii) bis zu 10 Aminosäurereste aufweist, die von der benachbarten intrazellulären Domäne, die an seinen C-Terminus gebunden ist, abgeleitet sind, wobei vorzugsweise das lösliche CD83-Protein aus den Aminosäureresten 20 bis 145 von SEQ ID Nr. 2 besteht; oder
(iii) funktionelle Sequenzen von bis zu 10 Aminosäureresten aufweist, die an seinen N-Terminus gebunden sind, und vorzugsweise das sCD83-Protein am N-Terminus die zusätzlichen Aminosäuren Gly-Ser-Pro-Gly oder Tyr-His-His-His-His-His-His trägt.

9. sCD83-Protein, funktionelles Derivat des sCD83-Proteins, dimere oder trimere Form des sCD83-Proteins oder dimere oder trimere Form des funktionellen Derivats des sCD83-Proteins zur Verwendung bei therapeutischem Haarwachstum gemäß Anspruch 7, wobei das sCD83-Protein ein Dimer oder Trimer ist, vorzugsweise ein Homodimer, das über einen oder mehrere der Cysteinreste der monomeren Form des sCD83-Proteins verbunden ist, am meisten bevorzugt das sCD83-Protein wie in Anspruch 8 definiert ist und/oder das Homodimer über den fünften Cysteinrest der monomeren Form des sCD83-Proteins verbunden ist.

10. sCD83-Protein, funktionelles Derivat des sCD83-Proteins, dimere oder trimere Form des sCD83-Proteins oder dimere oder trimere Form des funktionellen Derivats des sCD83-Proteins zur Verwendung bei therapeutischem Haarwachstum gemäß Anspruch 7, wobei das sCD83-Protein ein monomeres CD83-Protein ist, vorzugsweise ein monomeres CD83-Protein, bei dem einer oder mehrere der Cysteinreste durch gleiche oder verschiedene kleine und/oder polare Aminosäurereste, die aus Serin, Alanin und Glycin ausgewählt sind, substituiert sind, wobei am meisten bevorzugt
(i) das sCD83-Protein wie in den Ansprüchen 8 oder 9 definiert ist; oder
(ii) genau ein Cysteinrest, insbesondere der fünfte Cysteinrest, ersetzt ist; oder
(iii) das sCD83-Protein aus den Aminosäureresten 20 bis 144 von SEQ ID Nr. 2 besteht, wobei der Cysteinrest auf Position 129 durch einen Serinrest ersetzt ist; oder
(iv) das sCD83-Protein aus den Aminosäureresten 20 bis 144 von SEQ ID Nr. 2 besteht, wobei der Cysteinrest auf Position 129 durch einen Serinrest ersetzt ist und weiterhin am N-Terminus die zusätzlichen Aminosäuren Gly-Ser-Pro-Gly trägt.

11. sCD83-Protein, funktionelles Derivat des sCD83-Proteins, dimere oder trimere Form des sCD83-Proteins oder dimere oder trimere Form des funktionellen Derivats des sCD83-Proteins zur Verwendung bei therapeutischem Haarwachstum gemäß Anspruch 7, wobei das sCD83-Protein ein natives Glycosylierungsmuster aufweist oder nicht glycosyliert ist.

12. sCD83-Protein, funktionelles Derivat des sCD83-Proteins, dimere oder trimere Form des sCD83-Proteins oder dimere oder trimere Form des funktionellen Derivats des sCD83-Proteins zur Verwendung bei therapeutischem Haarwachstum gemäß einem der Ansprüche 7 bis 11, wobei das therapeutische Haarwachstum die systemische oder topische sCD83-Anwendung nach physischem Stress/Medikation und/oder Krankheit umfasst.

13. Zusammensetzung, umfassend eine lösliche Form eines Vertreters der CD83-Proteinfamilie, der aus der extrazellulären Domäne des CD83-Proteins mit den Aminosäureresten 20 bis 144 von SEQ ID Nr. 2 besteht (sCD83-Protein), funktionelles Derivat mit bis zu 10 Aminosäuren, die an den C- oder N-Terminus des sCD83-Proteins gebunden sind, und/oder bei dem bis zu 10 Aminosäuren des sCD83-Proteins substituiert sind, dimere oder trimere Form des sCD83-Proteins oder dimere oder trimere Form des funktionellen Derivats des sCD83-Proteins zur Verwendung bei therapeutischem Haarwachstum nach physischem Stress/Medikation und/oder Krankheit.

14. Zusammensetzung zur Verwendung bei therapeutischem Haarwachstum gemäß Anspruch 13, wobei
(i) das sCD83-Protein wie in den Ansprüchen 8 oder 11 definiert ist; und/oder
(ii) das therapeutische Haarwachstum die systemische oder topische sCD83-Anwendung nach physischem Stress/Medikation und/oder Krankheit umfasst.

15. Kosmetisches nichttherapeutisches Verfahren zur Beschleunigung des Haarwachstums und zur Haarpflege, wobei das Verfahren umfasst: die systemische oder topische Anwendung einer Zusammensetzung, die eine lösliche Form eines Vertreters der CD83-Proteinfamilie, der aus der extrazellulären Domäne des CD83-Proteins mit den Aminosäureresten 20 bis 144 von SEQ ID Nr. 2 besteht (sCD83-Protein), funktionelles Derivat mit bis zu 10 Aminosäuren, die an den C- oder N-Terminus des sCD83-Proteins gebunden sind, und/oder bei dem bis zu 10 Aminosäuren des sCD83-Proteins substituiert sind, dimere oder trimere Form des sCD83-Proteins oder dimere oder trimere Form des funktionellen Derivats des sCD83-Proteins umfasst, auf menschliche oder tierische Haut oder Haar.

16. Kosmetisches nichttherapeutisches Verfahren gemäß Anspruch 15, wobei das sCD83-Protein wie in den Ansprüchen 8 bis 11 definiert ist.

## Revendications

1. Forme soluble d'un membre de la famille de protéines CD83 constitué du domaine extracellulaire de la protéine CD83 avec les résidus d'acides aminés 20 à 144 de SEQ ID NO:2 (protéine sCD83), d'un dérivé fonctionnel de ladite protéine sCD83 ayant jusqu'à 10 acides aminés attachés à l'extrémité C- ou N-terminale de ladite protéine sCD83 et/ou ayant jusqu'à 10 acides aminés de ladite protéine sCD83 qui sont substitués, d'une forme dimère ou trimère de ladite protéine sCD83, ou d'une forme dimère ou trimère du dérivé fonctionnel de ladite protéine sCD83, pour l'utilisation dans la fermeture de plaies cutanées.

2. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la fermeture de plaies cutanées selon la revendication 1, où ladite protéine sCD83
(i) est constituée des résidus d'acide aminés 20 à 144 de SEQ ID NO:2 ; ou
(ii) possède jusqu'à 10 résidus d'acides aminés dérivés du domaine intracellulaire voisin, attachés à son extrémité C-terminale, de préférence la protéine CD83 soluble est constituée des résidus d'acides aminés 20 à 145 de SEQ ID NO:2 ; ou
(iii) possède des séquences fonctionnelles allant jusqu'à 10 résidus d'acides aminés attachés à son extrémité N-terminale, et de préférence ladite protéine sCD83 porte à l'extrémité N-terminale les acides aminés supplémentaires Gly-Ser-Pro-Gly ou Tyr-His-His-His-His-His-His.

3. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la fermeture de plaies cutanées selon la revendication 1, où la protéine sCD83 est un dimère ou un trimère, de préférence un homodimère relié par un ou plusieurs des résidus cystéine de la forme monomère de la protéine sCD83, le plus préférablement, la protéine sCD83 est telle que définie dans la revendication 2 et/ou l'homodimère est relié par le cinquième résidu cystéine de la forme monomère de la protéine sCD83.

4. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la fermeture de plaies cutanées selon la revendication 1, où la protéine sCD83 est une protéine CD83 monomère, de préférence, une protéine CD83 monomère où l'un des résidus cystéine, ou plusieurs d'entre eux, ont été substitués par les mêmes ou par des résidus d'acides aminés de petite taille et/ou polaires différents, choisis parmi la sérine, l'alanine et la glycine, le plus préférablement,
(i) la protéine sCD83 est telle que définie dans la revendication 2 ou 3 ; ou
(ii) un résidu cystéine, notamment le cinquième résidu cystéine, a été remplacé ; ou
(iii) la protéine sCD83 est constituée des résidus d'acides aminés 20 à 144 de SEQ ID NO:2, où le résidu cystéine en position 129 a été remplacé par un résidu sérine ; ou
(iv) la protéine sCD83 est constituée des résidus d'acides aminés 20 à 144 de SEQ ID NO:2, où le résidu cystéine en position 129 a été remplacé par un résidu sérine, et porte en outre à l'extrémité N-terminale les acides aminés supplémentaires Gly-Ser-Pro-Gly.

5. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la fermeture de plaies cutanées selon la revendication 1, où la protéine sCD83 a un schéma de glycosylation natif ou est non glycosylée.

6. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la fermeture de plaies cutanées selon l'une quelconque des revendications 1 à 5, où la cicatrisation des plaies comprend
(a) l'administration systémique de la protéine sCD83, du dérivé fonctionnel de ladite protéine sCD83, de la forme dimère ou trimère de ladite protéine sCD83, ou de la forme dimère ou trimère du dérivé fonctionnel de ladite protéine sCD83, pour la fermeture accélérée des plaies cutanées ; ou
(b) l'administration topique de la protéine sCD83, du dérivé fonctionnel de ladite protéine sCD83, de la forme dimère ou trimère de ladite protéine sCD83, ou de la forme dimère ou trimère du dérivé fonctionnel de ladite protéine sCD83, de préférence sous forme d'une solution de sCD83 à base de gel, pour la fermeture des plaies des lésions cutanées, y compris les plaies localement limitées et les plaies chroniques.

7. Forme soluble d'un membre de la famille de protéines CD83 constitué du domaine extracellulaire de la protéine CD83 avec les résidus d'acides aminés 20 à 144 de SEQ ID NO:2 (protéine sCD83), d'un dérivé fonctionnel ayant jusqu'à 10 acides aminés attachés à l'extrémité C- ou N-terminale de ladite protéine sCD83 et/ou ayant jusqu'à 10 acides aminés de ladite protéine sCD83 qui sont substitués, d'une forme dimère ou trimère de ladite protéine sCD83, ou d'une forme dimère ou trimère du dérivé fonctionnel de ladite protéine sCD83, pour l'utilisation dans la repousse thérapeutique des cheveux après un stress physique/un traitement et/ou une maladie.

8. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la repousse thérapeutique des cheveux selon la revendication 7, où ladite protéine sCD83
(i) est constituée des résidus d'acide aminés 20 à 144 de SEQ ID NO:2 ; ou
(ii) possède jusqu'à dix résidus d'acides aminés dérivés du domaine intracellulaire voisin, attachés à son extrémité C-terminale, de préférence la protéine CD83 soluble est constituée des résidus d'acides aminés 20 à 145 de SEQ ID NO:2 ; ou
(iii) possède des séquences fonctionnelles allant jusqu'à 10 résidus d'acides aminés attachés à son extrémité N-terminale, et de préférence ladite protéine sCD83 porte à l'extrémité N-terminale les acides aminés supplémentaires Gly-Ser-Pro-Gly ou Tyr-His-His-His-His-His-His.

9. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la repousse thérapeutique des cheveux selon la revendication 7, où la protéine sCD83 est un dimère ou un trimère, de préférence un homodimère relié par un ou plusieurs des résidus cystéine de la forme monomère de la protéine sCD83, le plus préférablement, la protéine sCD83 est telle que définie dans la revendication 8 et/ou l'homodimère est relié par le cinquième résidu cystéine de la forme monomère de la protéine sCD83.

10. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la repousse thérapeutique des cheveux selon la revendication 7, où la protéine sCD83 est une protéine CD83 monomère, de préférence, une protéine CD83 monomère où l'un des résidus cystéine, ou plusieurs d'entre eux, ont été substitués par les mêmes ou par des résidus d'acides aminés de petite taille et/ou polaires différents, choisis parmi la sérine, l'alanine et la glycine, le plus préférablement,
(i) la protéine sCD83 est telle que définie dans la revendication 8 ou 9 ; ou
(ii) un résidu cystéine, notamment le cinquième résidu cystéine, a été remplacé ; ou
(iii) la protéine sCD83 est constituée des résidus d'acides aminés 20 à 144 de SEQ ID NO:2, où le résidu cystéine en position 129 a été remplacé par un résidu sérine ; ou
(iv) la protéine sCD83 est constituée des résidus d'acides aminés 20 à 144 de SEQ ID NO:2, où le résidu cystéine en position 129 a été remplacé par un résidu sérine, et porte en outre à l'extrémité N-terminale les acides aminés supplémentaires Gly-Ser-Pro-Gly.

11. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la repousse thérapeutique des cheveux selon la revendication 7, où la protéine sCD83 a un schéma de glycosylation natif ou est non glycosylée.

12. Protéine sCD83, dérivé fonctionnel de ladite protéine sCD83, forme dimère ou trimère de ladite protéine sCD83, ou forme dimère ou trimère du dérivé fonctionnel de ladite protéine sCD83, pour l'utilisation dans la repousse thérapeutique des cheveux selon l'une quelconque des revendications 7 à 11, où dans la repousse thérapeutique des cheveux comprend l'application systémique ou topique de sCD83 après un stress physique/un traitement et/ou une maladie.

13. Composition comprenant une forme soluble d'un membre de la famille de protéines CD83 constitué du domaine extracellulaire de la protéine CD83 avec les résidus d'acides aminés 20 à 144 de SEQ ID NO:2 (protéine sCD83), d'un dérivé fonctionnel de ladite protéine sCD83 ayant jusqu'à 10 acides aminés attachés à l'extrémité C- ou N-terminale de ladite protéine sCD83 et/ou ayant jusqu'à 10 acides aminés de ladite protéine sCD83 qui sont substitués, d'une forme dimère ou trimère de ladite protéine sCD83, ou d'une forme dimère ou trimère du dérivé fonctionnel de ladite protéine sCD83, pour l'utilisation dans la repousse thérapeutique des cheveux après un stress physique/un traitement et/ou une maladie.

14. Composition pour l'utilisation dans la repousse thérapeutique des cheveux selon la revendication 13, dans laquelle
(i) la protéine sCD83 est telle que définie dans les revendications 8 à 11 ; et/ou
(ii) la repousse thérapeutique des cheveux comprend l'application systémique ou topique de sCD83 après un stress physique/un traitement et/ou une maladie.

15. Procédé cosmétique non thérapeutique pour l'accélération de la pousse des cheveux et pour les soins capillaires, lequel procédé comprend l'application par voie systémique ou topique d'une composition comprenant une forme soluble d'un membre de la famille de protéines CD83 constitué du domaine extracellulaire de la protéine CD83 avec les résidus d'acides aminés 20 à 144 de SEQ ID NO:2 (protéine sCD83), d'un dérivé fonctionnel de ladite protéine sCD83 ayant jusqu'à 10 acides aminés attachés à l'extrémité C- ou N-terminale de ladite protéine sCD83 et/ou ayant jusqu'à 10 acides aminés de ladite protéine sCD83 qui sont substitués, d'une forme dimère ou trimère de ladite protéine sCD83, ou d'une forme dimère ou trimère du dérivé fonctionnel de ladite protéine sCD83, sur la peau ou les cheveux humains ou sur la peau ou les poils des animaux.

16. Procédé cosmétique non thérapeutique selon la revendication 15, où la protéine sCD83 est telle que définie dans les revendications 8 à 11.
